# EUROPEAN PATENT SPECIFICATION

(11) **EP 3 920 887 B1**
(45) Date of publication and mention of the grant of the patent: **14.08.2024**
(21) Application number: 20752592.4
(22) Date of filing: 04.02.2020
(51) Int. Cl.: A61P 25/00, A61K 9/00, A61K 35/12, A61K 35/16, A61K 35/44, A61K 35/51, A61K 38/46

(54) **A PHOSPHATIDYLINOSITOL-GLYCAN-SPECIFIC PHOSPHOLIPASE D1 (GPLD1) POLYPEPTIDE FOR USE IN A METHOD OF TREATING OR PREVENTING AGE-RELATED COGNITIVE DYSFUNCTION**
EIN PHOSPHATIDYLINOSITOL-GLYCAN-SPEZIFISCHES PHOSPHOLIPASE D1 (GPLD1)-POLYPEPTID ZUR VERWENDUNG ZUR BEHANDLUNG ODER VORBEUGUNG ALTERSBEDINGTER KOGNITIVER DYSFUNKTION
POLYPEPTIDE PHOSPHOLIPASE D1 (GPLD1) SPÉCIFIQUE DE PHOSPHATIDYLINOSITOL-GLYCAN DESTINÉ À ÊTRE UTILISÉ DANS UNE MÉTHODE DE TRAITEMENT OU DE PRÉVENTION D'UN DYSFONCTIONNEMENT COGNITIF LIÉ À L'ÂGE.

(30) Priority: 04.02.2019 US 201962800944 P
(43) Date of publication of application: 15.12.2021
(73) Proprietor: The Regents of the University of California, Oakland CA 94607-5200 (US)
(72) Inventor: VILLEDA, Saul A., Oakland, California 94607-5200 (US); HOROWITZ, Alana, Oakland, California 94607-5200 (US); FAN, Xuelai, Oakland, California 94607-5200 (US)
(74) Representative: J A Kemp LLP
(86) International application number: PCT/US2020/016549
(87) International publication number: WO 2020/163306

(56) References cited:
- WO-A1-2019/164876
- WO-A1-96/05818
- US-A- 6 083 920
- US-A1- 2003 049 249
- US-A1- 2006 269 537
- US-A1- 2006 269 537
- US-A1- 2011 177 094
- HOROWITZ ALANA M. ET AL: "Blood factors transfer beneficial effects of exercise on neurogenesis and cognition to the aged brain", SCIENCE, vol. 369, no. 6500, 10 July 2020 (2020-07-10), US, pages 167 - 173, XP055919667, ISSN: 0036-8075, Retrieved from the Internet <URL:https://www.ncbi.nlm.nih.gov/pmc/articles/PMC7879650/pdf/nihms-1658458.pdf> DOI: 10.1126/science.aaw2622
- SEKI TSUNEYOSHI ET AL: "Galectin 3-binding protein suppresses amyloid-[beta] production by modulating [beta]-cleavage of amyloid precursor protein", JOURNAL OF BIOLOGICAL CHEMISTRY, vol. 295, no. 11, 29 January 2020 (2020-01-29), US, pages 3678 - 3691, XP055919903, ISSN: 0021-9258, DOI: 10.1074/jbc.RA119.008703
- SAUL A VILLEDA ET AL: "Young blood reverses age-related impairments in cognitive function and synaptic plasticity in mice", NATURE MEDICINE, vol. 20, no. 6, 4 May 2014 (2014-05-04), pages 659 - 663, XP055202055, ISSN: 1078-8956, DOI: 10.1038/nm.3569
- SMITH LUCAS K ET AL: "The systemic environment: at the interface of aging and adult neurogenesis", CELL AND TISSUE RESEARCH, SPRINGER, DE, vol. 371, no. 1, 9 November 2017 (2017-11-09), pages 105 - 113, XP036392829, ISSN: 0302-766X, [retrieved on 20171109], DOI: 10.1007/S00441-017-2715-8
- HOROWITZ ALANA M. ET AL: "Therapeutic potential of systemic brain rejuvenation strategies for neurodegenerative disease", F1000RESEARCH, vol. 6, 1 January 2017 (2017-01-01), pages 1291, XP055920253, Retrieved from the Internet <URL:https://f1000researchdata.s3.amazonaws.com/manuscripts/12349/f2bdee25-8843-41d0-b489-59338fe74d5f_11437_-_saul_villeda.pdf?doi=10.12688/f1000research.11437.1&numberOfBrowsableCollections=48&numberOfBrowsableInstitutionalCollections=4&numberOfBrowsableGateways=40> DOI: 10.12688/f1000research.11437.1
- FAN XUELAI ET AL: "Visual circuits for direction selectivity", ANNU. REV. NEUROSCI, 1 January 2017 (2017-01-01), pages 251 - 72, XP055920433, Retrieved from the Internet <URL:https://www.annualreviews.org/doi/pdf/10.1146/annurev-neuro-072116-031357> [retrieved on 20220512], DOI: 10.1146/annurev-neuro-072116
- SHIGEKI MASUDA, YUYA FUJISHIMA, NORIKAZU MAEDA, YURI TSUGAWA-SHIMIZU, YUTO NAKAMURA, YOSHIMITSU TANAKA, YOSHINARI OBATA, SHIRO FUK: "Impact of glycosylphosphatidylinositol-specific phospholipase D on hepatic diacylglycerol accumulation, steatosis, and insulin resistance in diet-induced obesity", AMERICAN JOURNAL OF PHYSIOLOGY: ENDOCRINOLOGY AND METABOLISM, vol. 316, no. 2, 20 November 2018 (2018-11-20), pages E239 - E250, XP055730361, DOI: 10.1152/ajpendo.00319.2018

## Description

Aging drives cognitive decline and increased risk for dementia-related neurodegenerative diseases. Reversing the effects of aging on the brain through systemic interventions such as exercise may offer a broad therapeutic approach (1-3), fulfilling an unmet need that is growing more pressing as the human population ages (4-6).

Exercise has long been reported in animal models to reverse age-related decline in adult neurogenesis, synaptic plasticity and cognitive function in the aged hippocampus (8-11), a brain region sensitive to the detrimental effects of aging (12). Similarly, it has been shown that systemic interventions, including heterochronic parabiosis (in which a young and old circulatory system are joined) and young blood plasma administration, rejuvenate regenerative capacity, synaptic plasticity and cognition in aged mice (13-18).

Horowitz et al., Science vol. 369, no. 6500, 10 July 2020, pages 167-173, discloses that blood factors transfer beneficial effects of exercise on neurogenesis and cognition to the aged brain. Seki et al., Journal of Biological Chemistry vol. 295, no. 11, 29 January 2020 pages 3678-3691 discloses that galectin 3- binding protein suppresses amyloid-β production by modulating β-cleavage of amyloid precursor protein. US 2006/269537 discloses the use of glycosylphosphatidylinositol specific phospholipase D (GPI-PLD) proteins in the treatment and diagnosis of diabetes. Villeda et al., Nature Medicine vol. 20, no. 6, 4 May 2014, pages 659-663, describes that exposure of an aged animal to young blood can counteract and reverse pre-existing effects of brain aging at the molecular, structural, functional and cognitive level. Smith et al., Cell and Tissue Research, vol. 371, no. 1, 9 November 2017, pages 105-113, describes how age-related changes in blood, blood-borne factors, and peripheral immune cells contribute to the age-related decline in adult neurogenesis in the mammalian brain. Horowitz et al., F1000Research, vol. 6, 1 January 2017, page 1291, describes blood-based rejuvenation strategies and their capacity to delay age-related molecular and functional decline in the aging brain. Fan et al., Annual Review of Neuroscience, 1 January 2017, pages 251-272, discloses the mechanisms of hippocampal aging and the potential for rejuvenation.

### SUMMARY OF THE INVENTION

The invention provides a phosphatidylinositol-glycan-specific phospholipase D1 (Gpld1) polypeptide that comprises the catalytic domain and has catalytic activity for use in a method of treating or preventing age-related cognitive dysfunction in an individual in need thereof, wherein the method comprises administering to the individual an effective amount of the Gpld1 polypeptide systemically or locally to the brain, thereby treating or preventing age-related cognitive dysfunction.

The invention further provides a nucleic acid construct encoding a Gpld1 polypeptide for use in a method of treating or preventing age-related cognitive dysfunction in an individual in need thereof, the method comprising introducing the nucleic acid construct into the patient.

The invention also provides genetically modified cells of an individual for use in a method of treating or preventing age-related cognitive dysfunction in the individual, the method comprising genetically modifying the cells *ex vivo* to enhance expression of a Gpld1 polypeptide using a genetic modification technique and administering the cells to the individual, optionally wherein the genetic modification technique is CRISPR/Cas or by introducing a nucleic acid construct encoding a Gpld1 polypeptide into the cells.

### FURTHER DISCLOSURE

The present disclosure is based, in part, on the discovery that glycosylphosphatidylinositol-specific phospholipase D1 (Gpld1) is an exercise-induced circulating blood factor in aging mice and healthy elderly humans; and moreover that increasing systemic Gpld1 levels induces rejuvenating effects on the hippocampus in aged mice.

Described herein is a method of treating or preventing age-related cognitive dysfunction in an individual in need thereof, the method comprising administering to the individual an effective amount of a phosphatidylinositol-glycan-specific phospholipase D1 (Gpld1) polypeptide, wherein the Gpld1 polypeptide is administered systemically or locally to the brain, thereby treating or preventing age-related cognitive dysfunction. In some aspects, the Gpld1 polypeptide is administered systemically. In some aspects, the Gpld1 polypeptide is administered by intravenous, intraperitoneal, subcutaneous, or intramuscular injection; administered orally or mucosally. In some aspects, the individual is a human. In some aspects, the human is at least 50 years old. The human may have mild cognitive impairment or dementia. In some aspects, the individual has Alzheimer's disease. Typically, the Gpld1 polypeptide has catalytic activity and comprises an amino acid sequence having at least 75%, or at least 95%, identity to amino acids 24-176 of SEQ ID NO:1. In some aspects, the Gpld1 polypeptide has catalytic activity and comprises an amino acid sequence having at least 75%, at least 80%, at least 90%, or at least 95% identity to the region of SEQ ID NO:1 that corresponds to the mature protein. In some aspects, the effective amount is 1 ug to 1000 ug per kg body weight of the individual. The Gpld1 polypeptide or a functional fragment thereof may be administered more than once as part of a course of treatment. In some aspects, the method further comprises testing the cognitive function of the individual after administering. For example, cognitive function is tested prior to administering the Gpld1 polypeptide and cognitive function of the individual after administration of the Gpld1 polypeptide is tested and compared to cognitive function prior to administering the polypeptide. In some aspects cognitive function is determined by testing the individual for semantic, episodic, procedural, priming, and/or working memory. Cognitive function may also be determined by testing the individual for language ability, executive function, visuospatial function, or dementia.

Described herein is a method of treating or preventing age-related cognitive dysfunction in an individual in need thereof, the method comprising introducing a nucleic acid construct encoding a Gpld1 polypeptide into the patient. In some aspects, the nucleic acid is introduced into the liver.

Described herein is a method of treating or preventing age-related cognitive dysfunction in an individual in need thereof, the method comprising genetically modifying cells, e.g., hepatocytes, of an individual to enhance expression of a Gpld1 polypeptide. In some aspects, the method comprises genetically modifying cells *ex vivo* and administering modified cells to the patient. In some aspects, the individual is a human. In some aspects, the individual has mild cognitive impairment or dementia. In some aspects, the individual has Alzheimer's disease.

### BRIEF DESCRIPTION OF THE DRAWINGS

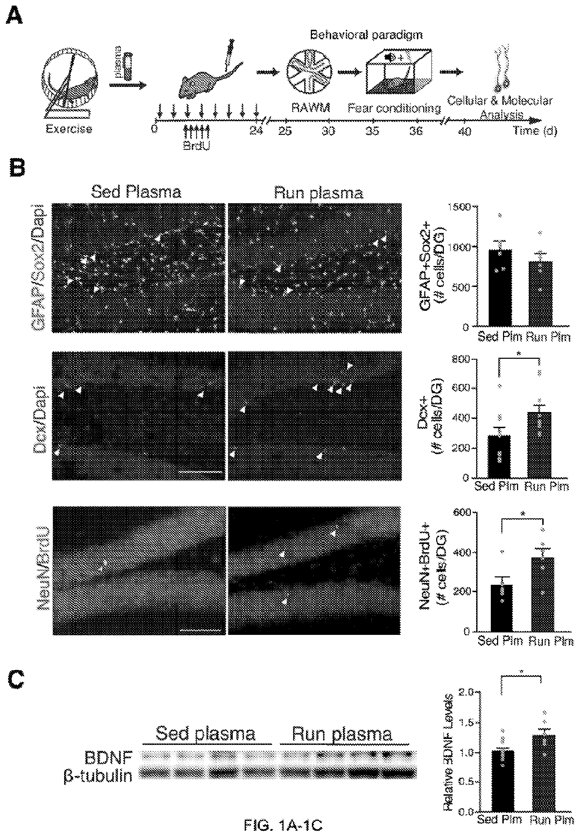

FIG. 1A-H. Systemic administration of exercise-induced circulatory blood **factors ameliorates impaired neurogenesis and cognition in the aged hippocampus.** (A) Blood plasma was collected from aged (18 months) exercised or sedentary mice and administered to sedentary aged mice 8 times over 24 days (100ul per intravenous injection). Schematic illustrates chronological order of aged exercise plasma administration and cognitive testing. (B) Representative field and quantification of GFAP/Sox2 double-positive, Dcx-positive, and NeuN/BrdU double-positive cells in the DG of the hippocampus of naive aged mice administered aged exercise or sedentary plasma (n=10-11 per group; Arrowheads point to individual cells; scale bars 100µm). (C) Western blot and quantification of BDNF in the hippocampus of naive aged mice administered aged exercise or sedentary plasma (n=6-10 per group). Quantification normalized to β-Tubulin. (D,E) Spatial learning and memory were assessed by radial arm water maze (RAWM) as number of entry errors committed during the training and testing phases. Overall learning and memory was analyzed between Block 1 and 10 (n=12-15 per group). (F-H) Associative fear memory was assessed using contextual (G) and cued (H) fear conditioning as percent time spent freezing 24 hours after training. Baseline freezing (F) was assessed as percent time spent freezing prior to fear conditioning (n=12-19 per group). All data are shown as mean±s.e.m.; *P<0.05, **P<0.01, ****P<0.0001 t test (B,C,F-H); repeated measures analysis of variance (ANOVA) with Bonferroni post-hoc test (D); ANOVA with Tukey's post-hoc test (E).

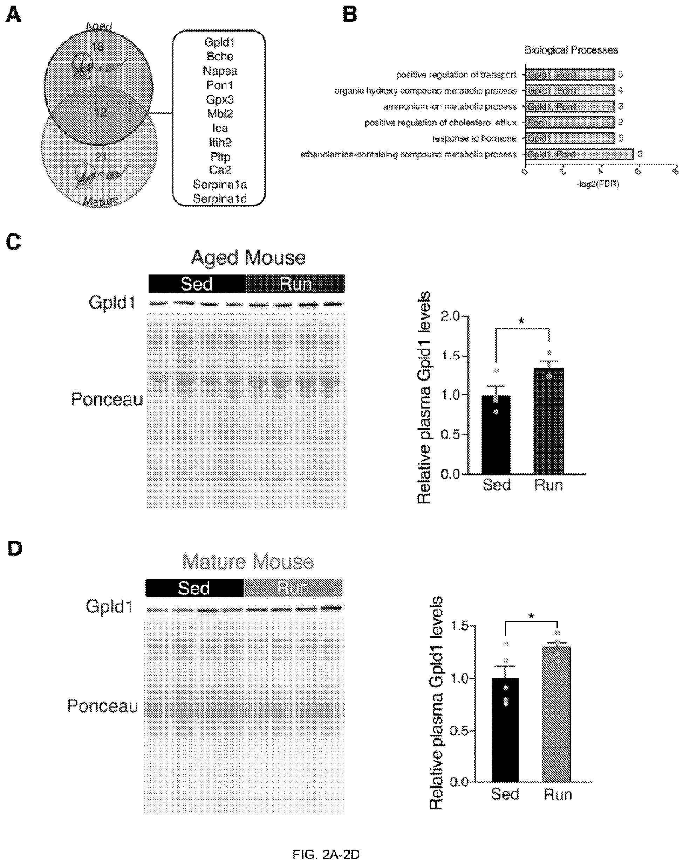

FIG. 2A-H. Exercise increases systemic levels of Gpld1 in mature and aged **mice and healthy elderly humans.** (A) Venn diagram of results from aged exercise and mature exercise proteomic screens. In blue are the number of proteins that increase with exercise in aged (18 months) mice, and in teal are proteins that increase with exercise in mature (7 months) mice. Listed are the 12 proteins that increase with exercise in both aged and mature mice. (B) Enrichment analysis of 12 proteins upregulated by exercise in mature and aged mice. Gpldl and Pon1 are listed next to the processes in which they are implicated. The number of proteins represented in each process is listed to the right of the bar. (C,D) Western blots with corresponding Ponceau S stains and quantification of Gpldl in equal volumes of blood plasma from aged (C) and mature (D) exercise and sedentary mice (n=4-5 per group). (E,F) Correlation between plasma Gpldl levels in aged exercise and sedentary mice and number of errors committed during the final block of RAWM (E) or time spent freezing in contextual fear conditioning (F). (G) Schematic illustrates activity tracking in healthy elderly (70-79 years) humans and criteria for categorization as active (>7100 steps per day) and sedentary (<7100 steps per day). (H) Western blot and quantification of Gpldl in equal volumes of blood plasma from active and sedentary healthy elderly humans (n=8-12 per group). All data are shown as mean±s.e.m.; *P<0.05, t test (C,D,H), linear regression (E,F).

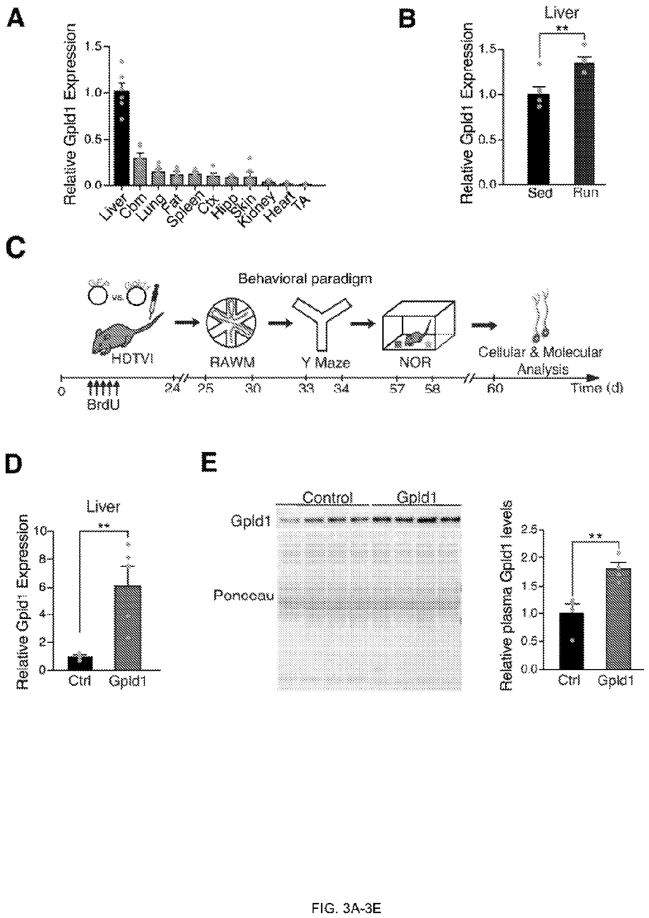

FIG. 3A-J. Increased systemic GPLD1 ameliorates impaired neurogenesis and **cognition in the aged hippocampus.** (A,B) Quantitative reverse-transcription PCR of Gpldl across tissues in sedentary mice (A), and in liver of aged exercised and sedentary mice (B). Gene expression relative to Gapdh (n=5-6 per group). (C) Aged (18 months) mice were given hydrodynamic tail vein injections (HDTVI) of expression constructs encoding either Gpldl or GFP control. Schematic illustrates chronological order of HDTVI, cognitive testing, and cellular and molecular analysis. (D) Quantitative reverse-transcription PCR of Gpldl in liver of aged mice expressing Gpldl or GFP control. Gene expression relative to Gapdh (n=5 per group). (E) Western blot with corresponding Ponceau S stain and quantification of Gpldl in equal volumes of blood plasma from aged mice expressing Gpldl or GFP control (n=4 per group). (F) Representative field and quantification of GFAP/Sox2 double-positive, Dcx-positive, and NeuN/BrdU double-positive cells in the DG of the hippocampus of aged mice expressing Gpldl or GFP control (n=6 per group; Arrowheads point to individual cells; scale bars 100µm). Quantification normalized to β-Tubulin. (G,H) Spatial learning and memory were assessed by radial arm water maze (RAWM) as number of entry errors committed during the training and testing phases. Overall learning and memory was analyzed between Block 1 and 10 (n=26 per group). (I) Spatial working memory was assessed by Y-Maze as time spent in the start, training, and novel arms during the testing phase (n=23-25 per group). (J) Object recognition memory was assessed by Novel Object Recognition (NOR) as time spent exploring a novel object 24 hours after training (n=8-12 per group). All data are shown as mean±s.e.m.; *P<0.05, **P<0.01, ***P<0.001, ****P<0.0001; t test (B,D,E,F,J); repeated measures analysis of variance (ANOVA) with Bonferroni post-hoc test (H); ANOVA with Tukey's post-hoc test (I,J); One-sample t test versus 50% (K).

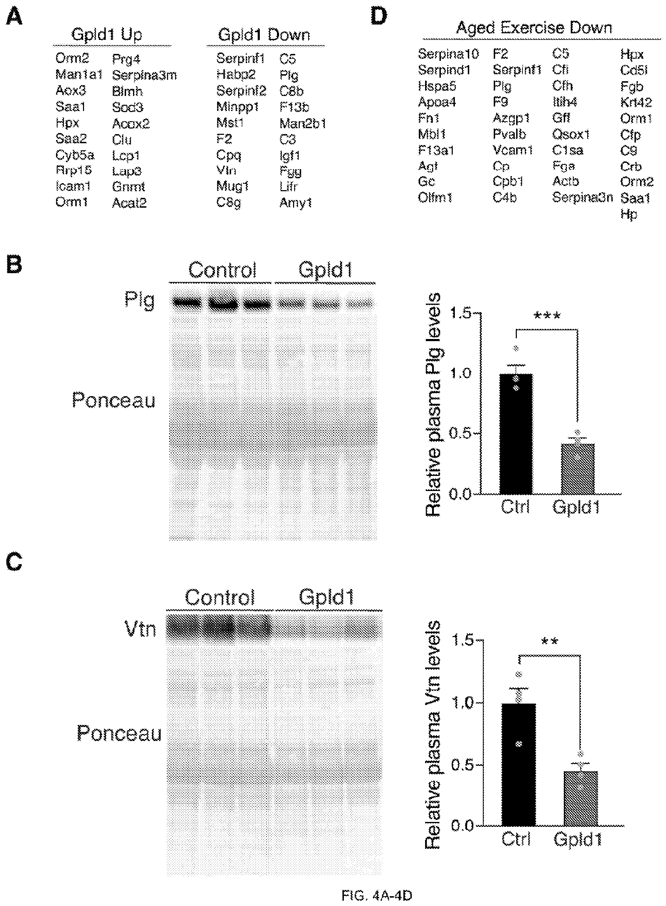

FIG. 4A-H. Increased systemic Gpld1 alters signaling cascades downstream of **GPI-anchored substrate cleavage in the aging systemic milieu.** (A) List of top 20 proteins up- and down-regulated in blood plasma of aged mice following Gpldl HDTVI compared to GFP HDTVI control, identified by mass spectrometry. (B-C) Western blot with corresponding Ponceau S stain and quantification of vitronectin (Vtn; A) and plasminogen (Plg; B) in equal volumes of blood plasma from aged mice 24 hours after HDTVI of Gpldl or GFP control (n=4 per group). (E,F) Enrichment analysis of plasma proteins downregulated with Gpld1 HDTVI (E) or exercise (F) in aged mice identified by mass spectrometry. The number of proteins represented in each process is listed to the right of each bar. (D) List of 41 proteins downregulated in blood plasma from aged mice following exercise. (G,H) Percent of proteins downregulated with Gpldl HDTVI (G, listed in A) or following exercise in aged mice (H, listed in D) that are liver-derived. Source tissue determined by Human Protein Atlas(24) and Tabula Muris(23). All data are shown as mean±s.e.m.; **P<0.01, ***P<0.001; t test (B, C).

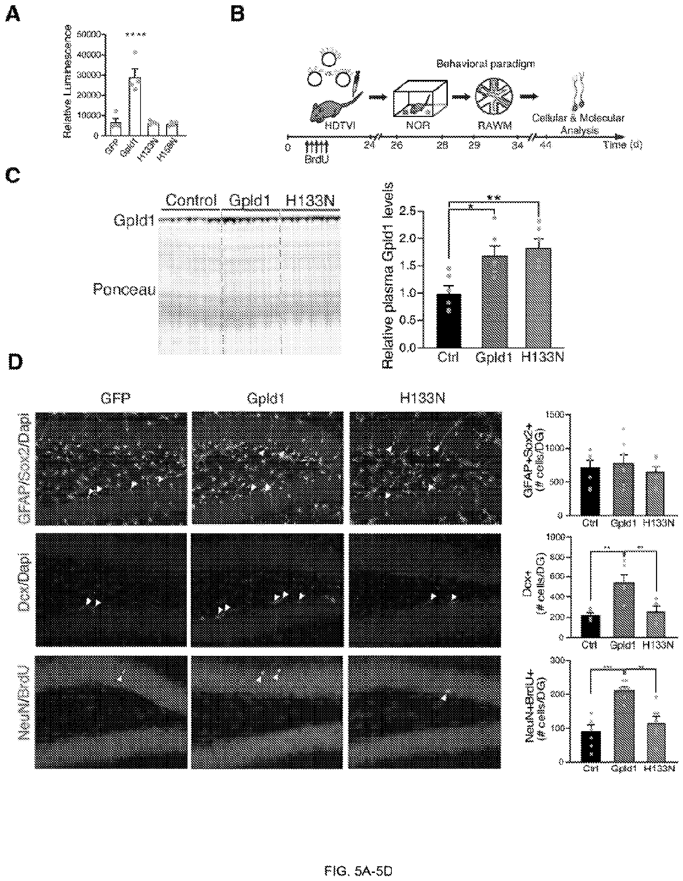

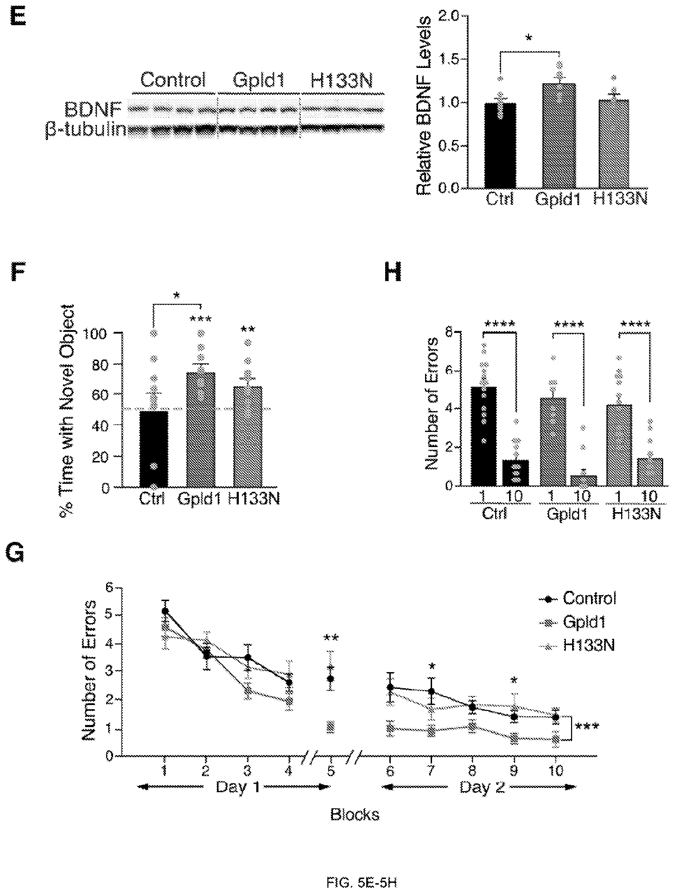

FIG. 5A-H. GPI-anchored substrate cleavage mediates the rejuvenating effects **of Gpld1 on the aged hippocampus.** (A) Luminescence-based quantification of alkaline phosphatase activity in cell culture supernatant 48 hours after transfection with Ubiquitin-lox-stop-lox-PLAP (GPI-anchored alkaline phosphatase) and EFla-Cre, in combination with either: CMV-GFP, CMV-Gpldl, CMV-H133N-Gpld1, or CMV-H158N-Gpld1 (n=3 samples/group). (B) Aged (18 months) mice were given hydrodynamic tail vein injections (HDTVI) of expression constructs encoding either Gpldl, catalytically dead H133N-Gpld1, or GFP control. Schematic illustrates chronological order of HDTVI, cognitive testing, and cellular and molecular analysis. (C) Western blot with corresponding Ponceau S stain and quantification of Gpldl in equal volumes of blood plasma from aged mice expressing Gpldl, Gpldl-H133N, or GFP control (n=6 per group). (D) Representative field and quantification of GFAP/Sox2 double-positive, Dcx-positive, and NeuN/BrdU double-positive cells in the DG of the hippocampus of aged mice expressing Gpldl, Gpld1-H133A, or GFP control (n=7 per group; Arrowheads point to individual cells; scale bars 100µm). (E) Western blot and quantification of BDNF in the hippocampus of aged mice expressing Gpldl, Gpld1-H133N, or GFP control (n=8 per group). Quantification normalized to β-Tubulin. (F) Object recognition memory was assessed by Novel Object Recognition (NOR) as time spent exploring a novel object 24 hours after training (n=9-1 1 per group). (G,H) Spatial learning and memory were assessed by radial arm water maze (RAWM) as number of entry errors committed during the training and testing phases. Overall learning and memory was analyzed between Block 1 and 10 (n=12-14 per group). All data are shown as mean±s.e.m.; *P<0.05, **P<0.01, ***P<0.001, ****P<0.0001; repeated measures analysis of variance (ANOVA) with Bonferroni post-hoc test (G); ANOVA with Tukey's post-hoc test (C-F, H); One-sample t test versus 50% (H).

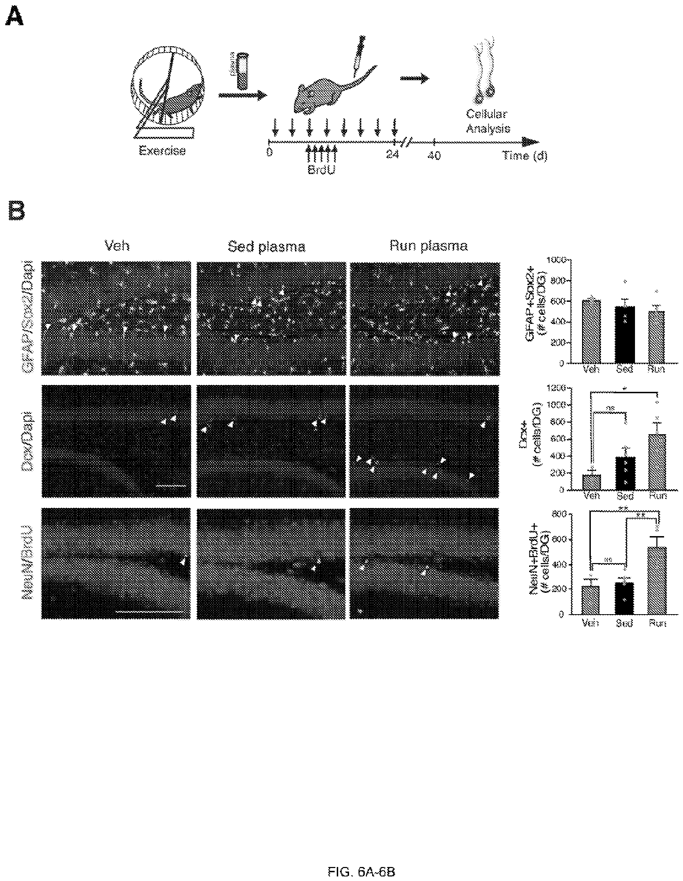

FIG. 6A-B. Systemic administration of exercise-induced circulating factors in **mature blood enhances adult hippocampal neurogenesis in aged mice.** (A) Blood plasma was collected from mature (7 months) exercised or sedentary mice and administered to sedentary aged mice 8 times over 24 days (100ul per intravenous injection). Schematic illustrates chronological order of mature exercise plasma administration and cellular analysis. (B) Representative field and quantification of GFAP/Sox2 double-positive, Dcx-positive, and NeuN/BrdU double-positive cells in the DG of the hippocampus of naive aged mice administered mature exercise or sedentary plasma (n=4-6 per group; arrowheads point to individual cells; scale bars 100µm). All data are shown as mean±s.e.m.; *P<0.05, **P<0.01; ANOVA with Tukey's post-hoc test (B).

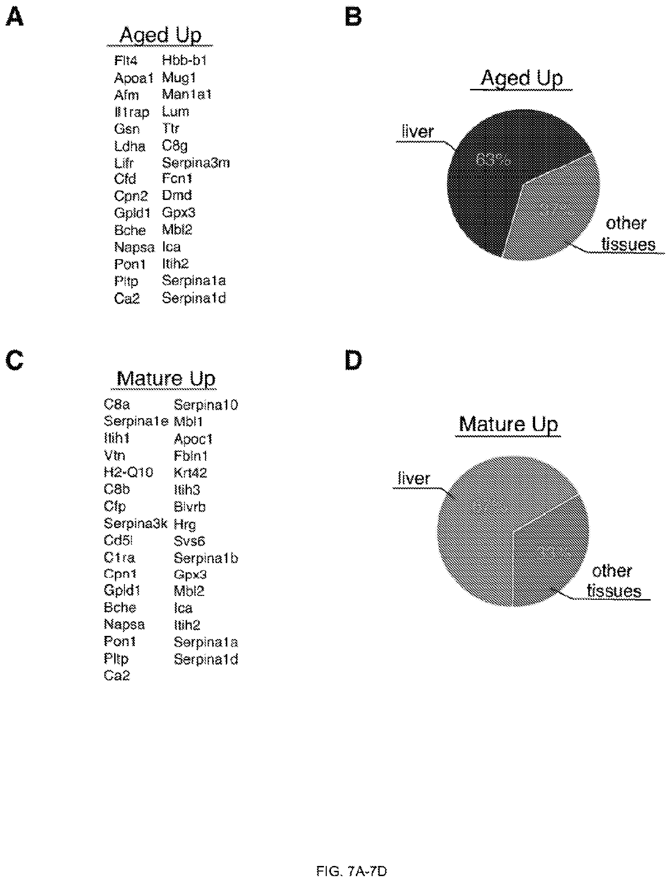

FIG. 7A-D. Proteomic analysis of exercise-induced circulating blood factors in **mature and aged mice.** (A) List of 30 proteins upregulated in blood plasma of aged mice with exercise. (C) List of 33 proteins upregulated in blood plasma of mature mice with exercise. (B,D) Percent of proteins upregulated following exercise in aged mice (B, listed in A) or mature mice (D, listed in C) that are liver-derived. Source tissue determined by Human Protein Atlas and Tabula Muris.

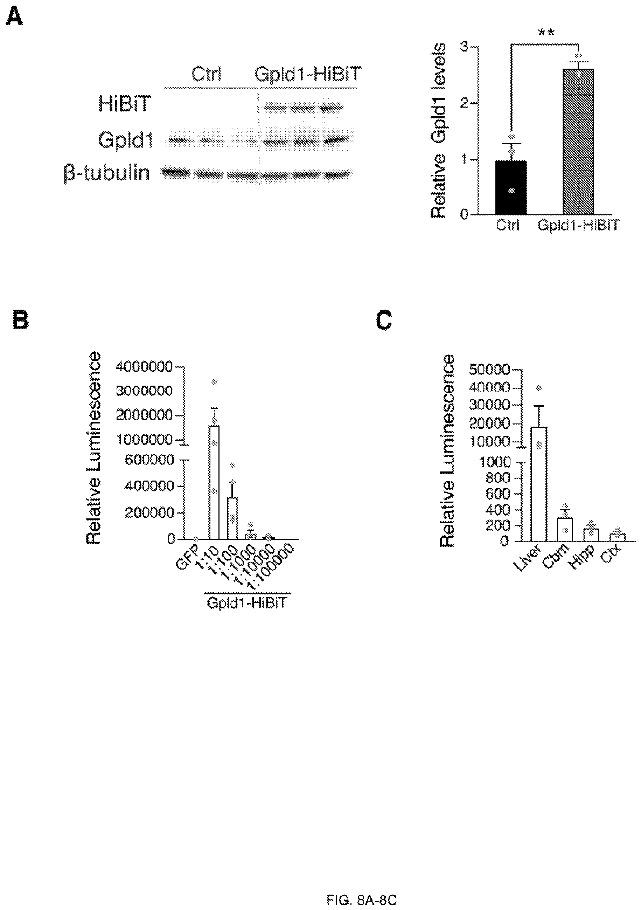

FIG 8A-C. Assessment of Gpld1 localization. (A) Western blot and quantification of Gpldl and HiBiT following transfection of control (GFP) or HiBiT-tagged Gpldl (Gpldl - HiBiT) constructs in 293T cells *in vitro.* (n = 3 per group) (B,C) Aged (23 months) mice were given hydrodynamic tail vein injections (HDTVI) of expression constructs encoding either Gpldl, Gpldl-HiBiT, or GFP control. Luminescence-based quantification of Gpldl-HiBiT in serial dilutions of blood plasma (B) or tissues (C) from aged mice following HDTVI (n = 3-4 per group). All data are shown as mean±s.e.m; **P<0.01; t test (A).

### DETAILED DESCRIPTION

### Terminology

As used herein, the term "Gpldl" refers to phosphatidylinositol-glycan-specific phospholipase D, which hydrolyzes the inositol phosphate linkage in proteins anchored by phosphatidylinositol glycans (GPI-anchor), which release the proteins from the membrane. Gpldl is classified under EC number 3.1.4.50. GPLD1 is a soluble protein with two functional domains, an N-terminal catalytic domain and a predicted C-terminal β-propeller domain. GPLD1 hydrolyzes GPI anchors that have acylated inositol. An illustrative human Gpldl polypeptide sequence is available under UniProt Accession Number P80108 and is provided as SEQ ID NO:1. Two isoforms produced by alternative splicing have been described. Isoform 1 (SEQ ID NO:1) has been deemed as the canonical sequence. Isoform 2 (identifier P80108-2) differs from SEQ ID NO:1 as follows: amino acids 165-176 are TVYLHLLNFLVV (instead of is GDVLSQFEFNFN as shown in SEQ ID NO:1) and residues 177-840 is missing. The NCBI isoform 1 reference sequences for human Gpldl are NP_001494.2 (amino acid) and NM-001503.4 (nucleic acid). Natural variants having single amino acid changes have also been described, e.g., at positions 17,(L>V), 30 (V>I), 275 (D>E), 350 (I>V), 396 (G>S), 461 (V>M), 694 (M>V), and 698 (T>I). Human Gpldl sequences include any polypeptide sequence encoded by a Gpldl gene, which has been localized to human chromosome 6p22. Mammalian Gpldl polypeptide sequences include those described for chimpanzee (XP_518268.4), rhesus monkey (XP_002803682.1), dog (XP_535902.2), cow (NP_777241.1), mouse (NP_032182.2) rat, (NP_001093982.1) as well as non-mammalian animals including chicken and frog. Conserved domains are described in HomoloGene: 1142 entry.

The terms "cognition," "cognitive ability," "cognitive function," and like terms refer to a collection of mental tasks and functions, including but not limited to: memory (e.g., semantic, episodic, procedural, priming, or working); orientation; language; problem solving; visual perception, construction, and integration; planning; organizational skills; selective attention; inhibitory control; and ability to mentally manipulate information.

The terms "improved cognition," "increased cognitive ability," "improved cognitive function," and like terms refer to an improvement in cognition under a given condition (*e.g*. treatment with Gpldl) compared to cognition absent that particular condition (*e.g*., absent treatment with Gpldl). For an individual experiencing cognitive decline, an improvement in cognition might be a reduction in the rate of cognitive decline *(i.e.,* an improvement compared to the absence of treatment), but not an actual improvement in cognitive ability. An increase in cognitive ability can also be an increase in brain activity in a specified area, *e.g.,* as determined by MRI, or an inhibition of brain activity that results in better overall brain function. An increase in cognitive ability can also be improvement in a cognitive performance test as described in more detail herein. An improvement or increase in cognitive ability can be in any one cognitive aspect or function, or any combination of individual cognitive functions.

An individual in need of improved cognitive function refers to individuals with age-related cognitive decline; a neurodegenerative disease; a mental or mood disorder; traumatic brain injury; developmental delay; genetic disorder resulting in reduced cognitive ability; brain injury due to stroke, brain cancer, MS, epilepsy, radiation or chemotherapy; etc. An individual in need of improved cognitive function can also include individuals that desire increased mental function to fight the effects of stress, sleep deprivation, jet lag, or pain, or to heighten ability for a particular task. A more complete and specific list of such individuals in included in the "Cognitive conditions and disorders" section herein.

A "subject," "patient" or "mammal" to be treated by the methods disclosed herein can mean either a human or non-human animal, such as a non-human primate, or non-primate mammal.

A "label" or a "detectable moiety" is a composition detectable by spectroscopic, photochemical, biochemical, immunochemical, chemical, or other physical means. For example, useful labels include ³²P, fluorescent dyes, electron-dense reagents, enzymes (*e.g*., as commonly used in an ELISA), biotin, digoxigenin, or haptens and proteins or other entities which can be made detectable, *e.g*., by incorporating a radiolabel into a polypeptide or antibody specifically reactive with a target polypeptide. Any method known for conjugating a protein to the label may be employed, *e.g*., using methods described in Hermanson, Bioconjugate Techniques 1996, Academic Press, Inc., San Diego.

A "labeled" molecule (*e.g*., Gpldl polypeptide) is one that is bound, either covalently, through a linker or a chemical bond, or noncovalently, through ionic, van der Waals, electrostatic, or hydrogen bonds to a label such that the presence of the molecule may be detected by detecting the presence of the label bound to the molecule.

The term "diagnosis" refers to a relative probability that a disorder is present in an individual. Similarly, the term "prognosis" refers to a relative probability that a certain future outcome may occur in the individual. For example, in the context of the present disclosure, prognosis can refer to the likelihood that an individual suffer cognitive decline, or the likely severity of the disease (*e.g*., severity of symptoms, rate of functional decline, etc.). The terms are not intended to be absolute, as will be appreciated by any one of skill in the field of medical diagnostics.

The terms "therapy," "treatment," and "amelioration" refer to any reduction in the severity of symptoms (cognitive decline), or improvement in cognitive function, or where motor function is affected, an improvement in motor function. As used herein, the terms "treat" and "prevent" are not intended to be absolute terms. Treatment and prevention can refer to any delay in cognitive decline, amelioration of symptoms (*e.g*., confusion), etc. Treatment and prevention can be complete or partial, such that cognition is better than would be expected without treatment (*e.g*., compared to cognition in the same individual before treatment or compared to cognition in similar non-treated individuals). The effect of treatment can be compared to an individual or pool of individuals not receiving the treatment, or to the same patient prior to treatment or at a different time during treatment. In some aspects, cognition is improved by at least 1%, as compared, *e.g*., to the individual before administration or to a control individual not undergoing treatment. In some aspects, cognition is improved by at least 2, 3, 5, 7, 10, 15, 20, 25%, 50%, 75%, 80%, or 90%, or more, determined using tests of cognition, molecular proxies, or structural changes associated with brain function.

The terms "effective amount," "effective dose," "therapeutically effective amount," etc. refer to that amount of the therapeutic agent sufficient to ameliorate a disorder, as described above. For example, for the given parameter, a therapeutically effective amount will show an increase or decrease of therapeutic effect at least 1%, 2%, 5%, 10%, 15%, 20%, 25%, 40%, 50%, 60%, 75%, 80%, 90%, or at least 100%. Therapeutic efficacy can also be expressed as "-fold" increase or decrease. For example, a therapeutically effective amount can have at least a 1.2-fold, 1.5-fold, 2-fold, 5-fold, or more effect over a control.

As used herein, the term "pharmaceutically acceptable" is used synonymously with physiologically acceptable and pharmacologically acceptable. A pharmaceutical composition will generally comprise agents for buffering and preservation in storage, and can include buffers and carriers for appropriate delivery, depending on the route of administration.

The terms "dose" and "dosage" are used interchangeably herein. A dose refers to the amount of active ingredient given to an individual at each administration. For the present disclosure, the dose refers to the amount of Gpldl polypeptide. The dose will vary depending on a number of factors, including frequency of administration; size and tolerance of the individual; type and severity of the condition; risk of side effects; and the route of administration. One of skill in the art will recognize that the dose can be modified depending on the above factors or based on therapeutic progress. The term "dosage form" refers to the particular format of the pharmaceutical, and depends on the route of administration. For example, a dosage form can be in a liquid, *e.g.,* a saline solution for injection.

"Subject," "patient," "individual" and like terms are used interchangeably and refer to, except where indicated, mammals such as humans and non-human primates, as well as dogs, horses, pigs, mice, rats, and other mammalian species. The term does not necessarily indicate that the subject has been diagnosed with a particular disease, but typically refers to an individual under medical supervision. A patient can be an individual that is seeking treatment, monitoring, adjustment or modification of an existing therapeutic regimen, etc.

The words "protein", "peptide", and "polypeptide" are used interchangeably to denote an amino acid polymer or a set of two or more interacting or bound amino acid polymers. The terms apply to amino acid polymers in which one or more amino acid residue is an artificial chemical mimetic of a corresponding naturally occurring amino acid, as well as to naturally occurring amino acid polymers, those containing modified residues, and non-naturally occurring amino acid polymer.

The term "amino acid" refers to naturally occurring and synthetic amino acids, as well as amino acid analogs and amino acid mimetics that function similarly to the naturally occurring amino acids. Naturally occurring amino acids are those encoded by the genetic code, as well as those amino acids that are later modified, *e.g*., hydroxyproline, γ-carboxyglutamate, and O-phosphoserine. Amino acid analogs refers to compounds that have the same basic chemical structure as a naturally occurring amino acid, *e.g.,* an α carbon that is bound to a hydrogen, a carboxyl group, an amino group, and an R group, *e.g*., homoserine, norleucine, methionine sulfoxide, methionine methyl sulfonium. Such analogs may have modified R groups (*e.g*., norleucine) or modified peptide backbones, but retain the same basic chemical structure as a naturally occurring amino acid. Amino acid mimetics refers to chemical compounds that have a structure that is different from the general chemical structure of an amino acid, but that functions similarly to a naturally occurring amino acid.

As to amino acid sequences, one of skill will recognize that individual substitutions, deletions or additions to a nucleic acid, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the disclosure. The following amino acids are typically conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (*see, e.g.*, Creighton, Proteins (1984)).

The terms "nucleic acid" and "polynucleotide" are used interchangeably herein to refer to deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, polypeptide-nucleic acids (PNAs). Unless otherwise indicated, a particular nucleic acid sequence also encompasses "conservatively modified variants" thereof (e.g., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem., 260:2605-2608 (1985); Rossolini et al., Mol. Cell. Probes, 8:91-98 (1994)). The term nucleic acid is used interchangeably with gene, cDNA, mRNA, oligonucleotide, and polynucleotide.

The terms "identical" or "percent identity," in the context two or more nucleic acid or polypeptide sequences, refer to two or more sequences or subsequences that are the same or have a specified percentage of nucleotides, or amino acids, that are the same (*i.e.,* about 60% identity, preferably at least 65%, 70%, 75%, 80%, or 85% identity; and often at least 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithm (*e.g*., a BLASTP algorithm with default parameters for comparison of two polypeptide sequences), or by manual alignment and visual inspection. *See e.g.,* the NCBI web site at ncbi.nlm.nih.gov/BLAST. Such sequences are then said to be "substantially identical." This definition also refers to, or may be applied to, the complement of a nucleotide test sequence. The definition also includes sequences that have deletions and/or additions, as well as those that have substitutions. As described below, the algorithms can account for gaps and the like. Gpldl polypeptides as employed in the present disclosure typically have at least that is at least 70%, 75%, 80%, or 85% identity; and often at least 90%, 91%, 92%, 93%, 94%, 95%, 96%, 97%, 98%, 99%, or higher identity over a specified region, *e.g.* 200, 400, 500, 600, 700, or 800 amino acids, or greater in length, or over the entire length of the reference sequence.

### Gpld1 polypeptides

As described above, Gpldl hydrolyzes the inositol phosphate linkage in proteins anchored by phosphatidylinositol glycans. Human Gpldl sequences are described above. Other mammalian Gpldl polypeptide sequences, including chimpanzee (Accession No. XP_518268.4), rhesus monkey (Accession No. XP_002803682.1), dog (Accession No. XP_535902.2), cow (Accession No. NP_777241.1), mouse (Uniprotein No. 070362) and rat (Uniprotein No. Q8R2H5); as well as non-mammalian animals including chicken and frog are available. An illustrative mouse Gpldl sequence is provided in SEQ ID NO:2. The region of mouse sequence SEQ ID NO:2 encoding the mature polypeptide has 79% identity and 87% positive when aligned to the amino acid of mature human Gpldl SEQ ID NO:1 (SEQ ID NO:1 as the query in an alignment using BLASTP).

Gpldl has an N-terminal catalytic domain. Histidines at positions 29, 125, 133, and 158, as determined with reference to SEQ ID NO:1, have been demonstrated to be important for catalytic activity as assessed in mice (see, *e.g.,* Heller et al., Eur. J. Biochem. 224:823-833, 1994 and Raikwar et al., Biochem J. 391:285-289, 2005). The N-terminal catalytic domain comprises the first 276 amino acids (amino acids 1-276). Of these, amino acids 1-23 are considered to be a signal peptide to target the immature protein to the endoplasmic reticulum prior to secretion. Amino acids 376-816 are comprise a C-terminal B-propeller domain. The N-terminal domain alone has demonstrated catalytic activity (Heller *et al., supra).* However, the C-terminal domain of Gpldl also modulates its catalytic activity, as deletion of terminal portions of the domain reduces enzymatic activity of Gpldl (Stadelmann, et. al., Biochimica et Biophysica Acta 1355:107-113, 1997.).

In some aspect, a Gpldl polypeptide described herein has at least 90%, or at least 90%, 91%, 92%, 93%, 94%, 95%, 96%, 97%, 98%, 99%, or higher, identity, over the mature polypeptide sequence of SEQ ID NO:1; or a region of SEQ ID NO:1, *e.g.,* of at least 300 amino acids in length, or at least 150, 180, 200, 250, 300, or 400 amino acids in length, comprising the Gpldl catalytic domain.

A functional variant or fragment of Gpldl is a variant or fragment that retains any Gpldl activity, e.g., at least 20%, 30%, 40%, 50%, 60%, 70%, 80%, 90%, or 100% of the level mature human Gpldl polypeptide. Such activities include activities as described. Additional activities include causing changes in magnetic resonance imaging (MRI) brain scans, e.g., functional MRI, electroencephalograph (EEG), and transcranial magnetic and electrical stimulation (TMS and TES); and improved performance in neuropsychologic testing and cognitive ability.

### Methods of Improving Cognitive Function

Gpldl polypeptides, or functional variants and fragments thereof, can be used to improve cognition for a number of conditions and situations. This includes treatment of individuals with lower than normal or declining cognitive ability, or prophylactic treatment of individuals in need of improved or increased cognitive ability. Gpldl polypeptide levels in an individual can be increased by administering a functional recombinant Gpldl polypeptide, by administering a nucleic acid that encodes a functional Gpldl polypeptide, *e.g*., as a viral or plasmid vector, or by genetic manipulation of cells in an individual, *e.g*., using CRISPR/CAS genetic modification techniques, or techniques comprising use of transcription activator-like effector nucleases (TALENS) or zinc finger nucleases.

Gpldl polypeptides (and functional variants and fragments thereof) and genetic manipulation to increase the levels of Gpldl polypeptide can be used to prevent or reduce cognitive decline associated with aging, *e.g.* in individuals 50 years of age or older, or 60 years of age or older, or upon initial signs of cognitive decline.

Gpldl polypeptides (and functional variants and fragments thereof) can also be used to treat individuals with age-related, non-age related, or disease related conditions including, but not limited to dementia and neurodegenerative diseases that may include impaired cognition, e.g., Alzheimer's disease, Parkinson's disease, Huntington's disease, frontotemporal dementia, progressive supranuclear palsy, corticobasalar degeneration, mild cognitive impairment, vascular dementia, Lewy body dementia, amyotropic lateral sclerosis, prion disorder, or HIV-related dementia.

In some aspects, an individual treated in accordance with the disclosure has Alzheimer's Disease or Mild Cognitive Impairment, frontotemporal dementia; or vascular dementia.

As used herein, "Alzheimer's disease" refers to senile dementia as diagnosed using commonly accepted criteria in the art, such as the criteria set forth by The National Institute of Neurological and Communicative Disorders and Stroke and the Alzheimer's disease and Related Disorders Association and/or the criteria as listed in the Diagnostic and Statistical Manual of Mental Disorders (DSM-IV-TR) published by the American Psychiatric Association. The Diagnostic and Statistical Manual of Mental Disorders (Fourth Edition, revised in 2000), also known as the DSM-IV-TR, outlines a detailed set of criteria for the diagnosis of Alzheimer's disease.

In some aspects, an individual may have mild to moderate dementia, or early-stage Alzheimer's disease, which can be identified using neurological testing and other clinical endpoints. For example, a subject with mild to moderate dementia, e.g., Alzheimer's disease, can be identified using the Mini-Mental State Examination (MMSE), Typically, a score of 16 to 26 (both inclusive) is indicative of mild to moderate Alzheimer's disease. Patients with advanced Alzheimer's disease can also be identified based on clinical parameters. Subjects with this form of Alzheimer's disease may no longer respond to therapy with acetylcholinesterase inhibitors, and may have a markedly reduced acetylcholine level.

In some aspects, a patient treated with a polypeptide of the disclosure may have Mild Cognitive Impairment. Such patients are at risk for development of Alzheimer's disease. Mild Cognitive Impairment can be diagnosed and evaluated using any of the many objective tests or criteria well-known and accepted in the fields of psychology or psychiatry.

"Frontotemporal dementia" is a neurodegenerative disease characterized by progressive neuronal loss predominantly involving the frontal and/or temporal lobes. It is distinguished from Alzheimer's disease and Lewy body dementia based on the fact that it does not manifest with amyloid plaques, neurofibrillary tangles, or Lewy bodies. The term "frontotemporal lobar degeneration" or "FTLD" is used to describe the specific pathological diseases that result in frontotemporal dementia syndromes. These are united by their impact on frontal and temporal brain structures. Subtyping is based on the specific proteins found within neuronal inclusions. Most degeneration subtypes are either FTLD-tau, which includes Pick's disease, CBD and PSP, all of which show tau-containing inclusions or FTLD-TDP, which includes several subtypes in which TDP-43 containing inclusions are seen.

In some aspects, a subject treated with a Gpldl polypeptide as described herein has a mental or mood disorder, *e.g*., depression, schizophrenia, attention deficit/ hyperactivity disorder, autism spectrum disorder, intellectual disability, a mood disorder, and a psychotic disorder.

In some aspects, a subject treated with a Gpldl polypeptide as described herein has a childhood neurodevelopmental syndrome or brain tumors, *e.g*., X-linked mental disability or retardation, astrocytoma, ependymoma, medulloblastoma, oligodendroglioma, Down's syndrome, Angelman's syndrome, Rett's syndrome; phenylketonuria, Lesch-Nyhan, galactosemia,or adrenoleukodystrophy.

In some aspects, a subject treated with a Gpldl polypeptide as described herein has a cognitive decline associated with chemotherapy and/or radiation therapy.

Additional conditions and disorders that can be treated with a Gpldl polypeptide include: pain-associated cognitive effects, traumatic brain injury, stroke, multiple sclerosis, neuroautoimmune disease, epilepsy, delirium, paraneoplastic disorder, developmental delay, and leukodystrophies.

Gpldl polypeptides (and functional variants and fragments thereof) can be also be administered to provide increased cognition for individuals desiring improved cognition, *e.g*., individuals exposed to stress, sleep deprivation, or jet lag, or for individuals requiring superior cognitive function, such as surgeons, air-traffic controllers, and military personal.

Cognitive ability can be measured using any method known in the art, *e.g.,* for testing memory, language ability, executive functions, visuospatial function, dementia, or multi-parameter neuropsychological abilities. In some aspects, Gpldl administration (as a polypeptide or by genetic manipulation) results in at least a 1%, 2%, 5%, 7%, 10%, 15%, 20%, 30%, 50%, or greater improvement in score on a standard cognitive ability test (*e.g.*, measured 1-3 days after administration). In some aspects, the testing is carried out more than once for an individual, e.g., one or more time over the course of treatment with Gpldl.

For example, standard tests for memory and learning can be applied, *e.g.,* to determine semantic, episodic, procedural, priming, and/or working (*i.e.,* short term) memory. Common tests include Cambridge prospective memory test (CAMPROMPT), memory assessment scales (MAS), Rey auditory verbal learning test, Rivermead behavioral memory test, Test of memory and learning (TOMAL), Wechsler memory scale (WMS), and Test of memory malingering (TOMM). Tests for language functions include, e.g., Boston Diagnostic Aphasia Examination (BDAE), Comprehensive aphasia test (CAT), and Multilingual aphasia examination (MAE).

Executive function (*e.g*., problem solving, planning, organization, inhibitory control) can be tested using Behavioral assessment of dysexecutive syndrome (BADS), CNS vital signs (Brief Core Battery), Controlled oral word association test (COWAT), Delis-Kaplan Executive Function System (D-KEFS), Digit vigilance test, Kaplan Baycrest neurocognitive assessment (KBNA), Hayling and Brixton tests, Tests of variables of attention (TOVA), Wisconsin card sorting test (WCST), or Test of everyday attention (TEA). Visuospatial ability (*e.g*., visual perception, construction and integration) can be tested using the Clock Test, Hooper visual organization task (VOT), or Rey-Osterrieth complex figure tests. Dementia can be quantified using the clinical dementia rating or dementia rating scale.

Multi-parameter tests for neuropsychological function (*e.g*., cognitive function) include but are not limited to the Barcelona neuropsychological test (BNT), Cambridge neuropsychological test automated battery (CANTAB), Cognistat, Cognitive assessment screening instrument (CASI), Cognitive function scanner (CFS), Dean-Woodcock neuropsychology assessment system (DWNAS), General practitional assessment of cognition (GPCOG) Mini mental state examination (MMSE), NEPSY, or the CDR computerized assessment system.

Alternatively, cognition can be determined using structural or molecular proxies for cognitive activity, *e.g*., compared over time to detect changes. Cognitive changes can be detected, *e.g*., by observing changes to brain structure, connectivity, activation, inhibition, or synaptic plasticity, *e.g*., by MRI, fMRI, EEG, TMS and TES, and/or any combination of these. In some aspects, brain activity is observed. In some aspects, Gpldl administration results in a 1.5-fold, 2-fold, 5-fold, 7-fold, 10-fold, or greater increase in brain activity (*e.g*., measured 24-30 days after administration). Molecular proxies for improved cognition include, but are not limited to: increased levels of GluN2B, increased GluN2B synaptic localization, increased NMDA receptor activation, and/or increased c-fos activation in the brain. These measures are particularly relevant to cognition. Such method can include, *e.g*., obtaining a sample of neuronal tissue or CSF from an individual and using standard assays to determine gene expression or activation.

Similarly, in mice and other non-human animals, cognitive ability can be tested with measures of executive function (working memory, attention, processing speed, set shifting), visiospatial learning and memory, object memory, pattern recognition, fear memory, passive avoidance memory, habituation, and novel object recognition, for example. Common tests include but are not limited to the Morris water maze, Barnes maze, radial arm water maze, y-maze, T-maze, and open field habituation. Brain imaging techniques are similarly applicable.

In some aspects, a Gpldl polypeptide is administered to an individual to improve motor function. Accordingly, a Gpldl polypeptide composition of the present disclosure is administered to a subject, *e.g*., a human subject, having impaired motor function for the treatment of the impaired motor function. For example, the subject has stroke to the brain or spinal cord (ischemic or hemorrhagic), neurodegenerative disease (Parkinson's disease, Lewy body dementia, multiple system atrophy, amyotropic lateral sclerosis, prion disorder, Huntington's disease, supranuclear palsy), Parkinsonism, traumatic brain injury, neuroinfectious brain lesions, multiple sclerosis and related autoimmune and demyelinating disease, spinal cord lesions (compressive, infectious, toxic or metabolic, autoimmune , oncologic), brain tumor, epilepsy, paraneoplastic disorder, neurodevelopmental disorder (mitochondrial, autosomal genetic), muscle disease (polymyositis, dermatomyositis, inclusion body myositis, infectious, endocrine, metabolic, toxic, congenital myopathy, congential muscular dystrophy, hereditary), neuropathies (Guillain-Barre syndrome, axonl and demyelinating, diabetic, toxic, metabolic, infectious, critical illness, entrapment), tick paralysis, myasthenia gravis, and spinal muscular atrophy. Changes in motor function can be assayed as known in the art. Illustrative motor function assays include but are not limited to electromyogram and nerve conduction studies, direct or device-assisted clinical testing of strength, tone, and muscle bulk, reflex examination, coordination examination, and gait analysis. Assays for testing etiologies causing deficits of motor function include but are not limited to magnetic resonance imaging of the central nervous system, muscle biopsy, nerve biopsy, and laboratory studies.

### Administration of Gpldl polypeptides

Described herein are methods of improving cognitive function and/or improving impaired motor function in an individual comprising administering an effective amount of a Gpldl polypeptide to the individual. In some aspects, the method of treatment comprises administering to an individual an effective amount of a Gpldl polypeptide comprising the catalytic domain, *e.g*., comprising amino acids 24-176, of SEQ ID NO:1; or comprising the region of SEQ ID NO:1 that correspond to the mature polypeptide, or a functional variant or fragment thereof. In some aspects, the treatment is prophylactic, *e.g*., to prevent cognitive decline associated with aging. In some aspects, the individual has been diagnosed with a cognitive disorder. In some aspects, the individual is receiving or has received therapy for a cognitive disorder or for a condition that is related to cognitive function (*e.g*., cognitive decline in response to chemotherapy). In some aspects, a Gpldl polypeptide is administered to an individual that has impaired motor function.

In some aspects, the method further comprises monitoring the individual for cognitive ability, either through a molecular proxy (*e.g*., changes in NMDA receptor or c-fos activation, or GluN2B levels in the brain, changes in MRI brain scans (*e.g*., functional MRI), changes in EEG, changes in TMS and TES, changes in neuropsychologic test scores, or tests of cognitive ability (*e.g.,* for learning, short or long term memory, executive functions, language ability, and visuospatial function). In some aspects, the individual is monitored using more than one of the above tests in any combination. In some aspects, the dose of the Gpldl polypeptide for each administration is determined based on the therapeutic progress of the individual, *e.g*., where a higher dose is administered if the individual is not responding sufficiently to therapy.

In some aspects, the Gpldl polypeptide is administered in a pharmaceutical composition with a physiologically (*i.e.,* pharmaceutically) acceptable carrier. The term "carrier" refers to a typically inert substance used as a diluent or vehicle for a diagnostic or therapeutic agent. The term also encompasses a typically inert substance that imparts cohesive qualities to the composition. Physiologically acceptable carriers can be liquid, *e.g.,* physiological saline, phosphate buffer, normal buffered saline (135-150 mM NaCl), water, buffered water, 0.4% saline, 0.3% glycine, glycoproteins to provide enhanced stability (*e.g*., albumin, lipoprotein, globulin, etc.), and the like. Since physiologically acceptable carriers are determined in part by the particular composition being administered as well as by the particular method used to administer the composition, there are a wide variety of suitable formulations of pharmaceutical compositions of the present disclosure (*See, e.g.,* Remington's Pharmaceutical Sciences, 17th ed., 1989).

The presently described compositions can be sterilized by conventional, well-known sterilization techniques or may be produced under sterile conditions. Aqueous solutions can be packaged for use or filtered under aseptic conditions and lyophilized, the lyophilized preparation being combined with a sterile aqueous solution prior to administration. The compositions can contain pharmaceutically acceptable auxiliary substances as required to approximate physiological conditions, such as pH adjusting and buffering agents, tonicity adjusting agents, wetting agents, and the like, *e.g*., sodium acetate, sodium lactate, sodium chloride, potassium chloride, calcium chloride, sorbitan monolaurate, and triethanolamine oleate. Sugars can also be included for stabilizing the compositions, such as a stabilizer for lyophilized antibody compositions.

Dosage forms can be prepared for mucosal (*e.g*., nasal, sublingual, vaginal, buccal, or rectal), parenteral (*e.g*., subcutaneous, intravenous, intramuscular, or intraarterial injection, either bolus or infusion), oral, or transdermal administration to a patient. Examples of dosage forms include, but are not limited to: dispersions; suppositories; ointments; cataplasms (poultices); pastes; powders; dressings; creams; plasters; solutions; patches; aerosols (*e.g*., nasal sprays or inhalers); gels; liquid dosage forms suitable for oral or mucosal administration to a patient, including suspensions (*e.g*., aqueous or non-aqueous liquid suspensions, oil-in-water emulsions, or a water-in-oil liquid emulsions), solutions, and elixirs; liquid dosage forms suitable for parenteral administration to a patient; and sterile solids (*e.g*., crystalline or amorphous solids) that can be reconstituted to provide liquid dosage forms suitable for parenteral administration to a patient.

Injectable compositions can comprise a solution of the Gpldl polypeptide suspended in an acceptable carrier, such as an aqueous carrier. Any of a variety of aqueous carriers can be used, *e.g*., water, buffered water, 0.4% saline, 0.9% isotonic saline, 0.3% glycine, 5% dextrose, and the like, and may include glycoproteins for enhanced stability, such as albumin, lipoprotein, globulin, etc. In some aspects, normal buffered saline (135-150 mM NaCl) is used. The compositions can contain pharmaceutically acceptable auxiliary substances to approximate physiological conditions, such as pH adjusting and buffering agents, tonicity adjusting agents, wetting agents, *e.g*., sodium acetate, sodium lactate, sodium chloride, potassium chloride, calcium chloride, sorbitan monolaurate, triethanolamine oleate, etc.

Formulations suitable for parenteral administration, such as, for example, by intraarticular (in the joints), intravenous, intramuscular, intradermal, intraperitoneal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. Injection solutions and suspensions can also be prepared from sterile powders, granules, and tablets. In some aspects, the composition is administered by intravenous infusion, topically, intraperitoneally, intravesically, or intrathecally. The Gpldl polypeptide formulation can be provided in unit-dose or multi-dose sealed containers, such as ampoules and vials.

The Gpldl polypeptide composition, alone or in combination with other suitable components, can be made into aerosol formulations ("nebulized") to be administered via inhalation. Aerosol formulations can be placed into pressurized acceptable propellants, such as dichlorodifluoromethane, propane, and nitrogen.

The pharmaceutical preparation can be packaged or prepared in unit dosage form. In such form, the preparation is subdivided into unit doses containing appropriate quantities of the active component, *e.g*., according to the dose of Gpldl polypeptide. The unit dosage form can be a packaged preparation, the package containing discrete quantities of preparation. The composition can, if desired, also contain other compatible therapeutic agents. In some aspects, the Gpldl polypeptide composition can be formulated in a kit for administration.

In some aspects, a pharmaceutical composition comprising a Gpldl polypeptide is administered orally. In some aspects, a pharmaceutical composition comprising a Gpldl polypeptide is administered mucosally, *e.g*., nasally. In some aspects, a pharmaceutical composition comprising a Gpldl polypeptide is administered by injection, *e.g*., subcutaneous, intraperitoneal, intravenous, or intramuscular. In some aspects, a pharmaceutical composition comprising a Gpldl polypeptide is administered by infusion, *e.g*., using a reservoir or osmotic minipump.

An example of administration of a pharmaceutical composition includes storing the Gpldl polypeptide at 10 mg/ml in sterile isotonic aqueous saline solution at 4°C, and diluting it in an appropriate solution for injection prior to administration to the patient. In some aspects, the Gpldl polypeptide composition can be administered by intravenous infusion over the course of 0.25-2 hours. In some aspects, the administration procedure is via bolus injection.

In therapeutic use, the Gpldl polypeptide can be administered at the initial dosage of about 0.1 µg/kg to about 1000 µg/kg daily and adjusted over time. A daily dose range of about 1 µg/kg to about 500 µg/kg, or about 10 µg/kg to about 100 µg/kg, or about 30 µg/kg to about 50 µg/kg can be used. The dosage is varied depending upon the requirements of the patient, the severity of the condition being treated, and the route of administration. For example, for injection of Gpldl polypeptide, the effective dose is typically in the range of 10-100 µg/kg, while for direct delivery to the central nervous system (CNS), the effective dosage is lower, *e.g.,* 5-30 µg/kg. For oral administration, the effective dose is higher, *e.g.,* in the range of 50-10,000 µg/kg (*e.g.*, 100µg/kg-2mg/kg). The dose is chosen in order to provide effective therapy for the patient. The dose may be repeated at an appropriate frequency which may be in the range of once or twice per day, once or twice per week to once every three months, depending on the pharmacokinetics of the Gpldl polypeptide composition (*e.g.,* half-life in the circulation) and the pharmacodynamic response (*e.g.,* the duration of the therapeutic effect).

Administration can be periodic. Depending on the route of administration, the dose can be administered, *e.g.,* once every 1, 3, 5, 7, 10, 14, 21, or 28 days or longer (*e.g.,* once every 2, 3, 4, or 6 months). In some cases, administration is more frequent, *e.g*., 2 or 3 times per day. The patient can be monitored to adjust the dosage and frequency of administration depending on therapeutic progress and any adverse side effects, as will be recognized by one of skill in the art.

Dosages can be empirically determined considering the type and severity of cognitive condition diagnosed in a particular patient. The dose administered to a patient, in the context of the present disclosure, should be sufficient to affect a beneficial therapeutic response in the patient over time. The size of the dose will also be determined by the existence, nature, and extent of any adverse side-effects that accompany the administration of any particular composition in a particular patient, as will be recognized by the skilled practitioner.

Thus in some aspects, additional administration is dependent on patient progress, *e.g.,* the patient is monitored between administrations. For example, after the first administration or round of administrations, the patient can be monitored for cognitive ability or for side effects, *e.g*., weakness, dizziness, nausea, etc.

In some aspects, the individual has a chronic condition, so that Gpldl is administered over an indefinite period, *e.g.,* for the lifetime of the patient. In such cases, administration is typically periodic. Diseases that are considered long-term or chronic include, but are not limited to Alzheimer's disease, and cognitive decline associated with other diseases, including Parkinson's disease, Huntington's disease, and chronic conditions such as with hypertension and heart disease.

In some aspects, the Gpldl polypeptide is linked to a stabilizing moiety such as PEG, glycosylation, or a liposome or other nanocarrier. US Patent Nos. 4,732,863 and 7892554 and Chattopadhyay et al. (2010) Mol Pharm 7:2194 describe methods for attaching a polypeptide to PEG, PEG derivatives, and nanoparticles (e.g., liposomes). Liposomes containing phosphatidyl-ethanolamine (PE) can be prepared by established procedures as described herein. The inclusion of PE provides an active functional site on the liposomal surface for attachment. In some aspects, the Gpldl polypeptide is linked to an affinity tag, *e.g.,* a histidine tag (*e.g.,* 4-16 histidine residues), streptavidin, or an antibody target.

The Gpldl polypeptide can also be formulated as a sustained-release preparation (*e.g.*, in a semi-permeable matrices of solid hydrophobic polymers (*e.g.*, polyesters, hydrogels (for example, poly (2-hydroxyethyl-methacrylate), or poly (vinylalcohol)), polylactides. The Gpldl polypeptide can be entrapped in a nanoparticle prepared, for example, by coacervation techniques or by interfacial polymerization, for example, hydroxymethylcellulose or gelatin microcapsules and poly- (methylmethacylate) microcapsules, respectively, in colloidal drug delivery systems (for example, liposomes, albumin microspheres, microemulsions, nanoparticles and nanocapsules) or in macroemulsions.

In some aspects, the Gpldl polypeptide is labeled, *e.g.,* for tracking in the body or *ex vivo.* The Gpldl polypeptide can be labeled any diagnostic agent known in the art, as provided, for example, in the following references: Armstrong et al., Diagnostic Imaging, 5th Ed., Blackwell Publishing (2004); Torchilin, V. P., Ed., Targeted Delivery of Imaging Agents, CRC Press (1995); Vallabhajosula, S., Molecular Imaging: Radiopharmaceuticals for PET and SPECT, Springer (2009). The diagnostic agent can be detected by a variety of ways, including as an agent providing and/or enhancing a detectable signal. Detectable signals include, but are not limited to, gamma-emitting, radioactive, echogenic, optical, fluorescent, absorptive, magnetic, or tomography signals. Techniques for imaging the diagnostic agent can include, but are not limited to, single photon emission computed tomography (SPECT), magnetic resonance imaging (MRI), optical imaging, positron emission tomography (PET), computed tomography (CT), x-ray imaging, gamma ray imaging, and the like. The terms "detectable agent," "detectable moiety," "label," "imaging agent," and like terms are used synonymously herein.

In some aspects, the label can include optical agents such as fluorescent agents, phosphorescent agents, chemiluminescent agents, and the like. Numerous agents (*e.g*., dyes, probes, labels, or indicators) are known in the art and can be used in the present disclosure. (*See, e.g.*, Invitrogen, The Handbook-A Guide to Fluorescent Probes and Labeling Technologies, Tenth Edition (2005)). Fluorescent agents can include a variety of organic and/or inorganic small molecules or a variety of fluorescent proteins and derivatives thereof. For example, fluorescent agents can include but are not limited to cyanines, phthalocyanines, porphyrins, indocyanines, rhodamines, phenoxazines, phenylxanthenes, phenothiazines, phenoselenazines, fluoresceins, benzoporphyrins, squaraines, dipyrrolo pyrimidones, tetracenes, quinolines, pyrazines, corrins, croconiums, acridones, phenanthridines, rhodamines, acridines, anthraquinones, chalcogenopyrylium analogues, chlorins, naphthalocyanines, methine dyes, indolenium dyes, azo compounds, azulenes, azaazulenes, triphenyl methane dyes, indoles, benzoindoles, indocarbocyanines, benzoindocarbocyanines, and BODIPY^{™} derivatives. Fluorescent dyes are discussed, for example, in U.S. Pat. No. 4,452,720, U.S. Pat. No. 5,227,487, and U.S. Pat. No. 5,543,295.

The label can also be a radioisotope, e.g., radionuclides that emit gamma rays, positrons, beta and alpha particles, and X-rays. Suitable radionuclides include but are not limited to ²²⁵Ac, ⁷²As, ²¹¹At, ¹¹B, ¹²⁸Ba, ²¹²Bi, ⁷⁵Br, ⁷⁷Br, ¹⁴C, ¹⁰⁹Cd, ⁶²Cu, ⁶⁴Cu, ⁶⁷Cu, ¹⁸F, ⁶⁷Ga, ⁶⁸Ga, ³H, ¹⁶⁶Ho, ¹²³I, ¹²⁴I, ¹²⁵I, ¹³⁰I, ¹³¹I, ¹¹¹In, ¹⁷⁷Lu, ¹³N, ¹⁵O, ³²P, ³³P, ²¹²Pb, ¹⁰³Pd, ¹⁸⁶Re, ¹⁸⁸Re, ⁴⁷Sc, ¹⁵³Sm, ⁸⁹Sr, ^{99m}Tc, ⁸⁸Y and ⁹⁰Y. In some aspects, radioactive agents can include ¹¹¹In-DTPA, ^{99m}Tc(CO)₃-DTPA, ^{99m}Tc(CO)₃-ENPy2, ^{62/64/67}Cu-TETA, ^{99m}Tc(CO)₃-IDA, and ^{99m}Tc(CO)₃triamines (cyclic or linear). In some aspects, the agents can include DOTA and its various analogs with ¹¹¹In, ¹⁷⁷Lu, ¹⁵³Sm, ^{88/90}Y, ^{62/64/67}Cu, or ^{67/68}Ga. In some aspects, a nanoparticle can be labeled by incorporation of lipids attached to chelates, such as DTPA-lipid, as provided in the following references: Phillips et al., Wiley Interdisciplinary Reviews: Nanomedicine and Nanobiotechnology, 1(1): 69-83 (2008); Torchilin, V.P. & Weissig, V., Eds. Liposomes 2nd Ed.: Oxford Univ. Press (2003); Elbayoumi, T.A. & Torchilin, V.P., Eur. J. Nucl. Med. Mol. Imaging 33:1196-1205 (2006); Mougin-Degraef, M. et al., Int'l J. Pharmaceutics 344:110-117 (2007).

In some aspects, the diagnostic agent can be associated with a secondary binding ligand or to an enzyme (an enzyme tag) that will generate a colored product upon contact with a chromogenic substrate. Examples of suitable enzymes include urease, alkaline phosphatase, (horseradish) hydrogen peroxidase and glucose oxidase. Secondary binding ligands include, *e.g*., biotin and avidin or streptavidin compounds as known in the art.

### Administration of Gpld1 nucleic acids/Genetic manipulation

In certain aspects, nucleic acids encoding a Gpldl polypeptide is used for enhancing expression of Gpldl *in vivo.* These nucleic acids can be inserted into any of a number of well-known vectors, including viral expression vectors or plasmid-based vectors for the transfection of target cells and organisms. The nucleic acids may be transfected into cells, *e.g.,* hepatocytes, *ex vivo* or *in vivo* or administered *in vivo.*

In some aspects, the nucleic acid constructs encoding a Gpldl polypeptide is administered as a purified nucleic acid molecule, for example, as a DNA plasmid-based vectors ("naked" DNA). Alternatively, in some aspects, a nucleic acid construct encoding a Gpldl polypeptide may be contained within a viral vector and administered as a virus. Viral delivery systems include adenovirus vectors (*e.g*., Ad2, Ad5, Ad7), adeno-associated viral vectors, herpes simplex viral vectors, retroviral vectors, pox viral vectors (such as vaccinia and avian poxvirus vectors, such as the fowlpox and canarypox vectors), lentiviral vectors, alphavirus vectors, poliovirus vectors, and other positive and negative stranded RNA viruses, viroids, and virusoids, or portions thereof. Methods of constructing and using such vectors are well known in the art.

Nucleic acids for administration to a subject are formulated for pharmaceutical administration. While any suitable carrier known to those of ordinary skill in the art may be employed in the pharmaceutical compositions of this disclosure, the type of carrier will vary depending on the mode of administration. For parenteral administration, including intranasal, intradermal, subcutaneous or intramuscular injection or electroporation, the carrier may comprises water, saline, and optionally an alcohol, a fat, a polymer, a wax, one or more stabilizing amino acids or a buffer. General formulation technologies are known to those of skill in the art.

DNA immunogenic compositions can be administered once or multiple times as needed to induce the desired response (*e.g*., increased circulating levels of Gpldl). Multiple administrations can be administered, for example, bi-weekly, weekly, bi-monthly, monthly, or more or less often, as needed, for a time period sufficient to achieve the desired response.

Nucleic acids are administered by methods well known in the art, *e.g*., injection, electroporation, and the like. Typical delivery routes include parenteral administration, *e.g*., intradermal, intramuscular or subcutaneous routes. In some aspects, nucleic acids are introduced into cells, *e.g.,* hepatocytes, *ex vivo* and the cells are then introduced into the individual.

Nucleic acids can be administered in solution (*e.g*., a phosphate-buffered saline solution) by injection, usually by an intravenous, subcutaneous or intramuscular route. Dosages depend on the route of administration and can be readily determined by one of skill in the art.

In some aspects, cells of an individual are genetically modified using modification techniques, such as CRISPR/CAS to increase circulating levels of Gpldl polypeptides. For example, hepatocytes from an individual are genetically modified using a CRISPR-Cas9 to enhance expression of Gpldl.

### EXAMPLES

These examples demonstrate that systemic administration of blood plasma from exercised mice can reverse molecular, cellular and cognitive hallmarks of hippocampal aging. Gpldl was identified as an exercise-induced circulating blood factor sufficient to induce rejuvenating effects on the hippocampus of aged mice.

### Systemic blood plasma administration transfers the rejuvenating effects of exercise on the aged hippocampus.

The effect of direct exercise on the aged hippocampus was evaluated. As a control, we assessed age-related cellular and cognitive impairments in the hippocampus of aged (18 months) compared to young (3 months) mice. Subsequently, an independent cohort of aged mice was given continuous access to a running wheel for six weeks, while age-matched sedentary control mice were provided with alternative enrichment. Consistent with published reports (8,19), direct exercise resulted in increased adult neurogenesis, increased expression of Brain-derived neurotrophic factor (BDNF) and improved hippocampal-dependent learning and memory (data not shown) in aged mice.

Next, we tested whether the benefits of exercise on the aged hippocampus could be transferred through administration of exercise-induced circulating blood factors. Following direct exercise, blood was collected and plasma isolated from aged exercised and sedentary mice and pooled by group. An independent cohort of naive aged mice was then intravenously injected with aged exercise or sedentary plasma eight times over three weeks (FIG. 1A). We analyzed adult neurogenesis by immunohistochemical analysis. While no difference in the number of Sox2/GFAP neural progenitor cells was observed (FIG. 1B), we detected an increase in the number of Doublecortin (Dcx)-positive newly born neurons in the dentate gyrus region of the hippocampus in aged animals administered aged exercise plasma compared to sedentary plasma (FIG. 1B). We assessed neuronal differentiation and survival using a long-term BrdU incorporation paradigm, in which mature differentiated neurons express both BrdU and the neuronal marker NeuN. Aged mice administered with aged exercise plasma showed an increase in the number of mature neurons expressing both BrdU and NeuN in the dentate gyrus (FIG. 1B). Lastly, we examined expression of BDNF by Western blot and observed an increase in hippocampal expression following aged exercise plasma administration (FIG. 1C). Together, these data indicate that systemic administration of aged exercise plasma can transfer the beneficial effect of exercise on regenerative capacity in the aged hippocampus.

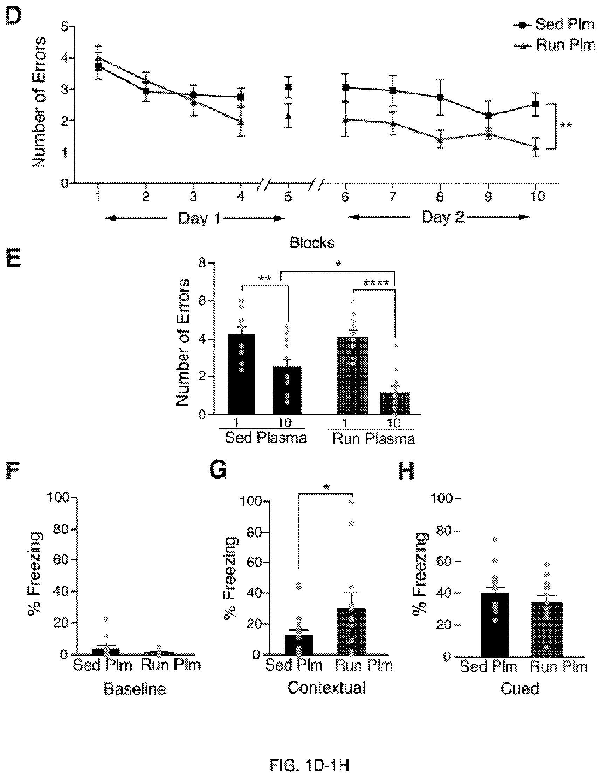

To assess the potential of aged exercise plasma to rescue age-related impairments in hippocampal-dependent learning and memory, we used radial arm water maze (RAWM) and contextual fear conditioning paradigms (FIG. 1A). In the training phase of the RAWM paradigm all mice showed similar spatial learning capacity (FIG. 1D). Remarkably, aged animals administered aged exercise plasma demonstrated improved learning and memory for the platform location during the testing phase of the task compared to aged sedentary plasma treated controls (FIG. 1D,E). During fear conditioning training, all mice exhibited similar baseline freezing regardless of treatment (FIG. 1F). However, aged mice receiving aged exercise plasma demonstrated increased freezing in contextual (FIG. 1G), but not cued (FIG. 1H) memory testing. These data indicate that exercise-induced circulating blood factors in aged plasma can ameliorate impairments in hippocampal-dependent learning and memory in aged mice.

Exercise has been consistently shown to enhance regenerative capacity in animal models ranging from young adult (*20-22*) to old ages (*8-11*)*.* Correspondingly, we investigated whether the beneficial effects of exercise observed in mice at younger ages could also be transferred to aged mice through circulating blood factors. We performed an independent study in which plasma derived from mature (6-7 months) exercised or sedentary mice was administered to aged mice. As a control, we examined the effect of direct exercise on the hippocampus of mature mice. In agreement with previous studies, we observed that direct exercise promotes neurogenic and cognitive enhancements in the hippocampus of mature mice. Following direct exercise, blood was collected and plasma isolated from mature exercised and sedentary mice and plasma was pooled by group. Naive aged mice were subsequently intravenously injected with mature exercise or sedentary plasma (FIG. 6A). account for any potential benefit of young blood that may persist at mature ages, an additional aged control group was administered saline. No significant changes were observed between aged mice administered mature sedentary plasma or saline. However, administration of mature exercise plasma resulted in increased adult neurogenesis compared to controls (FIG. 6B). These data indicate that exercise-induced circulating blood factors across ages can confer the benefits of exercise on the aged hippocampus.

### Gpldl is a liver-derived exercise blood factor that ameliorates age-related regenerative and cognitive decline.

Given that blood plasma from both aged and mature exercised mice rescued age-related impairments in the hippocampus, we sought to gain mechanistic insight as to individual circulating blood factors mediating these effects. To identify such factors, we employed a proteomic approach using isobaric tagging together with liquid chromatography tandem mass spectrometry, in which relative levels of soluble proteins were measured in the aged and mature exercised and sedentary plasma. Comparison of aged exercise and sedentary plasma identified 30 factors that increased after exercise (FIG. 2A, FIG. 7A), while comparison between mature exercise and sedentary plasma identified 33 factors that increased after exercise (FIG. 2A, FIG. 7C). Utilizing Tabula Muris (23) and the human protein atlas (24), we identified expression of these factors predominantly in the liver (FIG. 7B,D). Notably, 12 factors - Gpldl, Bche, Napsa, Pon1, Gpx3, Mbl2, Ica, Itih2, Pltp, Ca2, Serpinala and Serpinald - were elevated in both aged and mature exercise plasma (FIG. 2A). Functional enrichment analysis of these factors using STRING identified largely metabolic processes, in which Gpldl and Serum paraoxonase 1 (Pon1) were overrepresented (FIG. 2B). Correspondingly, to initiate functional investigations of individual exercise-induced blood factors we first selected Gpldl, a glycosylphosphatidylinositol degrading enzyme (25) not previously linked to aging, neurogenesis or cognition.

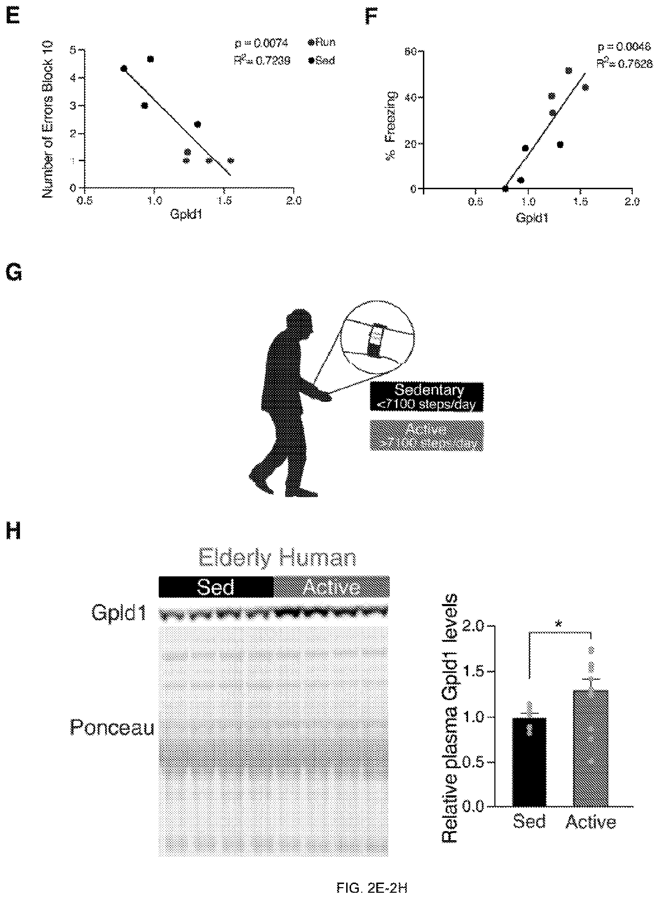

Relative levels of Gpldl were confirmed to increase in blood plasma of aged (FIG. 2C) and mature (FIG. 2D) exercised mice compared to age-matched sedentary controls by Western blot. In aged exercise and sedentary mice, we observed a significant correlation between increased Gpldl plasma levels and improved cognitive performance in the RAWM and contextual fear conditioning behavioral paradigms (FIG. 2E,F). Furthermore, we detected an increase in Gpldl in plasma from active compared to sedentary healthy elderly human individuals (FIG. 2G,H). These data identify Gpldl as an exercise-induced circulating blood factor in aged mice and humans with potential relevance to cognitive function.

To identify the potential source of exercise-induced systemic Gpldl, we first characterized Gpldl mRNA expression in mice across tissues that included liver, lung, spleen, skin, kidney, heart, muscle, cortex, hippocampus and cerebellum (FIG. 3A). We detected highest Gpldl expression in the liver (FIG. 3A), consistent with previous reports identifying the liver as the primary source of circulating Gpldl (26). Subsequently, we examined whether Gpldl mRNA expression changed in the liver as a function of aging, exercise or blood plasma administration. While no changes in Gpldl expression were detected during aging or following blood plasma administration, we observed increased Gpldl expression in the liver of aged exercised mice compared to sedentary animals (FIG. 3B). As a control, we also evaluated Gpldl expression in muscle and hippocampus and observed no changes under any condition. These data further identify Gpldl as a liver-derived exercise-induced circulating factor in aged mice.

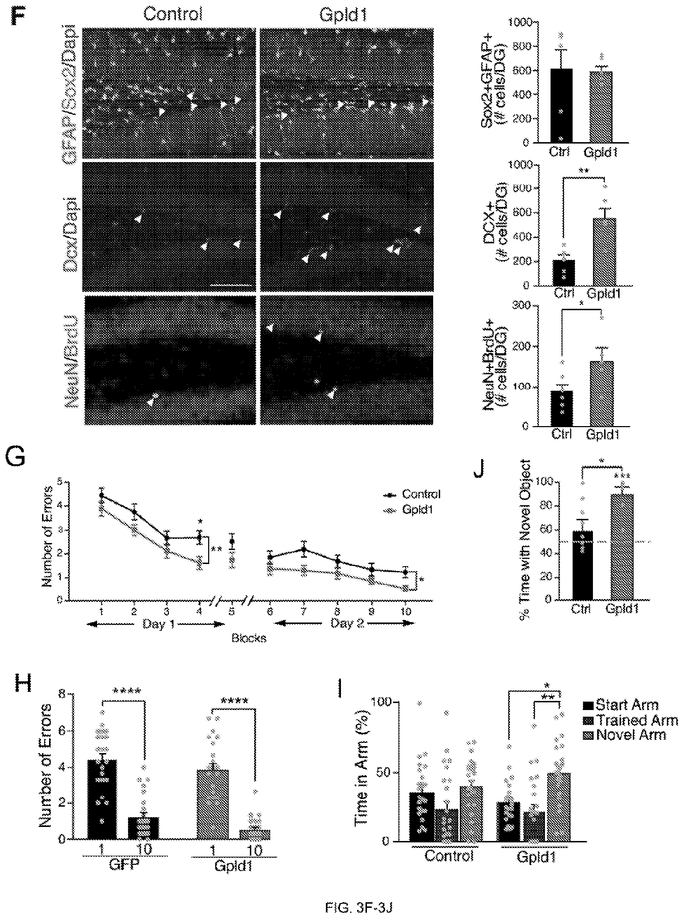

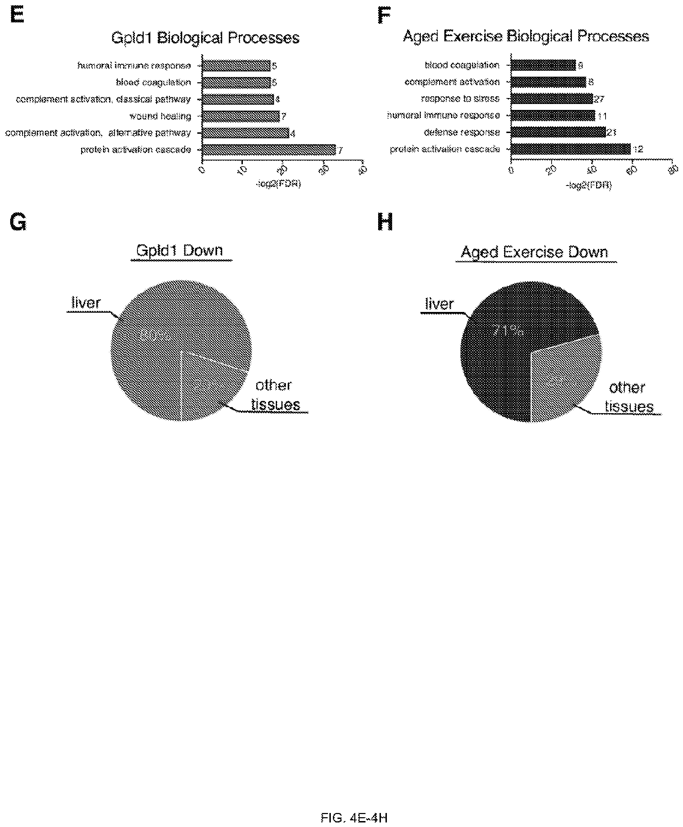

To test the functional consequence of increasing liver-derived systemic Gpldl on the aged hippocampus, we used a hydrodynamic tail vein injection (HDTVI)-mediated *in vivo* transfection approach to overexpress Gpldl in the liver. Aged mice were injected with expression constructs encoding either Gpldl or GFP control, and analysis was performed in a timeframe consistent with exercise plasma experiments (FIG. 3C). Increased Gpldl mRNA expression in the liver and elevated Gpldl blood plasma levels were confirmed following HDTVI in aged mice (FIGS. 3D, 4E). Using immunohistochemistry and Western blot analysis, we detected an increase in adult neurogenesis (FIG. 3F) and expression of BDNF in the hippocampus of aged mice with increased systemic Gpldl compared to controls. To assess the effect of systemic Gpldl on hippocampal-dependent learning and memory, we used RAWM, forced alteration Y maze and novel object recognition paradigms (FIG. 3C). It was observed that aged animals with increased systemic Gpldl committed significantly fewer errors in locating the target platform during the RAWM training and testing phases (FIG. 3G,H) compared to controls. During Y maze and novel object recognition testing, aged mice with increased systemic Gpldl spent significantly more time in the novel arm (FIG. 3I) and with the novel object (FIG. 3J). Lastly, we explored whether increasing Pon1, the second liver-derived circulating factor overrepresented in our exercise proteomic functional enrichment analysis (FIG. 2B), ameliorated age-related impairments in hippocampal-dependent cognitive function. Aged mice were given HDTVI with expression constructs encoding either Pon1 or GFP control; however, no cognitive improvements were observed in a timeframe consistent with Gpldl experiments. Together, these data indicate that selectively increasing liver-derived systemic Gpldl is sufficient to rejuvenate adult neurogenesis and cognitive function in the aged hippocampus.

### Coagulation and complement signaling cascades are altered in the aging systemic milieu downstream of Gpldl.

Next, we sought to delineate central versus peripheral mechanisms of action of liver-derived systemic Gpldl. To evaluate the potential of systemic Gpldl to cross the blood-brain-barrier we generated expression constructs encoding a HiBiT-tagged version of Gpldl (FIG. 8A). HiBiT is a small peptide with high affinity to LgBiT with which it forms a complex that produces a luminescent signal (27), allowing for sensitive and quantitative detection of tagged proteins. Aged mice were given HDTVI with expression constructs encoding HiBiT Gpldl, and elevated blood plasma levels were confirmed (FIG. 8B). We characterized HiBiT activity in mice across tissues that included plasma, liver, cortex, hippocampus and cerebellum (FIG. 8B,C). Luminescent signal was detected in plasma and liver (FIG. 8B,C). However, only negligible signal was detected in the brain (FIG. 8C), several orders of magnitude less than in plasma. These data indicate that liver-derived systemic Gpldl does not readily enter the brain, and point to a predominantly peripheral mechanism of action.

In its canonical role, Gpldl hydrolyzes GPI anchors that have acylated inositol, releasing membrane bound GPI-anchored proteins from the cell surface (*28-31*)*.* While the physiological role of Gpldl is poorly understood, cleavage of its substrates has been shown to regulate downstream signaling cascades important in biological processes, such as differentiation and inflammation (*30, 32*)*.* Therefore, we examined whether increasing liver-derived systemic Gpldl altered signaling cascades downstream of Gpldl substrate cleavage in the aging systemic milieu. We measured relative levels of soluble proteins in the plasma of aged mice given HDTVI with expression constructs encoding Gpldl or GFP control using label free mass spectrometry. We surveyed the top 20 up- and down-regulated proteins (FIG. 4A) for known signaling cascades associated with previously identified Gpldl substrates (30), and detected changes in the urokinase-type plasminogen activator receptor (uPAR) signaling pathway. uPAR is a Gpldl GPI-anchored substrate, whose proteolytic function regulates the plasminogen (Plg) activation system involved in coagulation, while its non-proteolytic function regulates extracellular matrix proteins through interactions with Vitronectin (Vtn) *(32, 33)*. We observed decreased levels of both Pin and Vtn in blood plasma of aged animals with increased systemic Gpldl compared to controls by Western blot (FIG. 4B,C). Additionally, we surveyed our previous exercise proteomic analysis for proteins that decrease in plasma of aged mice after exercise and also identified Plg (FIG. 4D). To further examine mechanistic synergies between increased systemic Gpldl versus exercise in the systemic milieu, we compared functional enrichment analysis using STRING of factors identified to decrease in aged plasma following either Gpldl overexpression (FIG. 4A) or exercise (FIG. 4D). Consistent with changes in the uPAR signaling pathway, we identified biological processes involved in coagulation, as well as the complement system, under both conditions (FIG. 4E,F). Additionally, of the factors surveyed following increased systemic Gpldl and exercise, we identified expression mostly in the liver using Tabula Muris (23) and the human protein atlas(24) (FIG. 4G,H). Together, these data posit systemic changes in signaling cascades downstream of GPI-anchored substrate cleavage as mediators of the rejuvenating effects of Gpldl and exercise.

### GPI-anchored substrate cleavage is necessary for the rejuvenating effects of Gpldl on the aged hippocampus.

Last, we tested whether the enzymatic activity of liver-derived systemic Gpldl, and subsequent GPI-anchored substrate cleavage, directly mediates its rejuvenating effects on adult neurogenesis and cognitve function in the aged hippocampus. Previous reports have demonstrated that the catalytic activity of Gpldl is dependent on histidines at position 133 and 158, with mutations in either site abrogating the enzymatic activity (*34*). We generated expression constructs encoding Gpldl with site-directed mutations converting histidine into asparagine, and abrogation of GPI-anchored substrate cleavage was validated *in vitro* (FIG. 5A). Aged mice were given HDTVI with expression constructs encoding either Gpldl, catalytically dead H133N Gpldl or GFP control (FIG. 5B), and elevated blood plasma levels were confirmed (FIG. 5C). We observed increased adult neurogenesis (FIG. 5D), increased BDNF expression (FIG. 5E), and cognitive improvements in the RAWM and novel object recognition tasks (FIG. 5F-H) in aged mice with increased systemic Gpldl compared to GFP control. However, no differences were observed in aged mice with increased systemic catalytically dead H133N Gpldl (FIG. 5D-H). These data indicate that the enzymatic activity of liver-derived systemic Gpldl is necessary for its rejuvenating effects on the aged hippocampus, and further highlight signaling cascades downstream of GPI-anchored substrate cleavage as molecular mediators of these rejuvenating effects.

### Summary and discussion of experimental results

Cumulatively, these data demonstrated that benefits of exercise on the aged brain can be transferred through blood-based interventions aimed at rejuvenating regenerative and cognitive function. Moreover, we identified the liver-derived exercise blood factor Gpldl, and signaling cascades downstream of its GPI-anchored substrate cleavage activity, as promising therapeutic targets. Ultimately, these data identify a liver-brain-rejuvenation axis by which circulating blood factors confer the rejuvenating effects of exercise in old age.

Currently, adult neurogenesis in humans remains controversial (35). Nevertheless, the most recent studies report adult neurogenesis in the human hippocampus through the ninth decade of life, with age-related decline exacerbated in Alzheimer's disease (AD) patients (*36*) and correlating with cognitive dysfunction (*37*). In the context of dementia-related neurodegenerative diseases, exercise is correlated with reduced risk for cognitive decline in the elderly, improves cognition in populations at-risk for AD, and is associated with better neurobehavioral outcomes even in autosomal dominant AD (*38-40*)*.* Similarly, exercise has been shown to ameliorate impairments in learning and memory in animal models of AD (*41, 42*) by increasing adult neurogenesis and levels of BDNF in the aged hippocampus(*42*) - benefits that we show are transferred through viable blood-based interventions. Taken together with the experimental data presented herein, these studies raise the exciting possibility that the beneficial effects of administering exercise blood factors may extend beyond normal aging towards reversing regenerative and cognitive decline in those suffering from age-related neurodegenerative disorders, such as AD.

An intimate relationship between changes in the aging systemic milieu and age-related regenerative and cognitive decline in the aging brain (13, *43-45*) has previously been described. These studies underscore increasing pro-youthful and decreasing pro-aging blood factors as strategies to reverse aging phenotypes. The proteomics analysis described above of proteins that decrease in plasma of aged mice following increased liver-derived Gpldl or exercise, now point to systemic changes in signaling cascades downstream of GPI-anchored substrate cleavage as mediators of brain rejuvenation. These data identify down regulation of the uPAR signaling pathway - and associated changes in the coagulation and complement system cascades - as potential pro-aging molecular targets. While the rejuvenating effects of liver-derived Gpldl and exercise are likely the result of changes in multiple signaling cascades in the aging systemic milieu, a prominent role is quickly emerging for the coagulation and complement pathways in aging. Indeed, changes in the coagulation pathway have now been identified as part of the senescence-associated secretory phenotype (SASP) (*46*), while blood-derived complement C1q promotes age-related regenerative decline in peripheral tissues (*47*). Interestingly, the benefits of targeting members of the coagulation pathway downstream of Gpldl have been recently reported in the context of neurodegeneration (*48*). Genetic mouse models deficient for Plg were protected from demyelination and paralysis in a mouse model of multiple sclerosis (*49*). Moreover, targeting blood-derived Plg through oligonucleotide technologies decreased amyloid β plaque deposition and neuropathology in a mouse model of AD (*48*). Given that systemic interventions eliciting neurogenic rejuvenation (*3, 12*) commonly promote rejuvenation in peripheral tissues(*50-52*), the data presented herein further support that t the beneficial effects of exercise could be promoted broadly across tissues through circulating blood factors.

### MATERIALS AND METHODS FOR EXAMPLES

**Animal Models.** The following mouse lines were used: C57BL/6 aged mice (The Jackson Laboratory), C57BL/6 aged mice (National Institutes of Aging), and C57BL/6 mature and aged mice (Taconic Biosciences). All studies were performed with male mice. The numbers of mice used to result in statistically significant differences was calculated using standard power calculations with α = 0.05 and a power of 0.8. We used an online tool (http://www.stat.uiowa.edu/~rlenth/Power/index.html) to calculate power and samples size based on experience with the respective tests, variability of the assays and inter-individual differences within groups. Within each individual experiment animals from the same strain and vendor that were aged together were utilized. All animals from Taconic and Jackson were acquired at 2 months of age and aged in-house for the duration of their life. Animals are moved to a new location for behavioral assessment. The two locations where we conducted behavior are on the UCSF Parnassus campus: the UCSF Rehabilitation Behavior Core and the Villeda Lab Behavioral Suite. Mice were housed under specific pathogen-free conditions under a 12 h light-dark cycle and all animal handling and use was in accordance with institutional guidelines approved by the University of California San Francisco IACUC.

**Animal Exercise.** Exercised mice were single-housed and given continuous access to a running wheel (Cat# ENV-047, Med Associates, Inc.) in their cage for 42 days. Distance ran per mouse was tracked using Wheel Manager software (Med Associates, Inc.). Mice evaluated in behavior assays had continuous access to running wheels for full duration of experiments. Sedentary mice were single-housed and give a house and nestlet as alternative enrichment.

**Animal Plasma collection and administration.** Mouse plasma was collected by intracardial bleed at time of sacrifice from 30 mature exercised, 30 mature sedentary, 30 aged exercised, and 30 aged sedentary mice. Plasma was prepared from blood collected with EDTA followed by centrifugation at 1000 X g. Plasma was maintained from individual mice for western blot analysis and mass spectrometry, or pooled for plasma administration. Plasma was aliquoted stored at -80°C until use. Prior to administration plasma was dialyzed using 3.5-kDa D-tube dialyzers (EMD Millipore) in PBS to remove EDTA. Aged mice were systemically treated with plasma (100ul/injection) isolated from mature exercised, mature sedentary, aged exercised, or aged sedentary mice via intravenous tail vein injection 8 times over 24 days.

**BrdU administration.** For BrdU labeling, 50mg/kg of BrdU (Sigma-Aldrich) was injected into mice intraperitoneally once a day for 5 days.

**Hydrodynamic Tail Vein Injection.** Hydrodynamic tail vein injections were performed as previously described(*53*). Endotoxin-free plasmids were prepared using the Qiagen Maxi-Prep Plus Kit (Cat# 12963, VWR). GPLD1 or GFP plasmid DNA (50ug) was suspended in 3mL saline and injected in the tail vein in 5-7 seconds in aged mice.

**Radial arm water maze.** Spatial learning and memory was assessed using the radial arm water maze (RAWM) paradigm according to established protocol(*54*). In this task, the mouse is trained to the location of a constant goal arm throughout the training and testing phase. The start arm changes each trial. Entry into an incorrect arm is scored as an error, and errors are averaged over training blocks (three consecutive trials). During training (day 1), mice are trained for 12 trails (blocks 1-4), with trials alternating between a visible and hidden platform. Following an hour break, learning is tested for 3 trials (block 5) using only a hidden platform. During testing (day 2), mice are tested for 15 trials (blocks 6-10) with a hidden platform. Investigators were blinded to treatment when scoring.

**Contextual fear conditioning.** In this task, mice learned to associate the environmental context (fear conditioning chamber) with an aversive stimulus (mild foot shock; unconditioned stimulus, US) enabling testing for hippocampal- dependent contextual fear conditioning. To assess amygdala-dependent cued fear conditioning, the mild foot shock was paired with a light and tone cue (conditioned stimulus, CS). Freezing behavior was used as a readout of conditioned fear. Specific training parameters are as follows: tone duration is 30 seconds; level is 70 dB, 2 kHz; shock duration is 2 seconds; intensity is 0.6 mA. This intensity is not painful and can easily be tolerated but will generate an unpleasant feeling. On the training day (day 1), each mouse was placed in a fear-conditioning chamber and allowed to explore for 2 min before delivery of a 30-second tone (70 dB) and light, ending with a 2-second foot shock (0.6 mA). Two minutes later, a second CS-US pair was delivered. On the testing day (day 2), each mouse was first placed in the fear-conditioning chamber containing the same exact context, but with no CS or foot shock. Freezing was analyzed for 1-2 minutes. One hour later, the mice were placed in a new context containing a different odor, cleaning solution, floor texture, chamber walls and shape. Animals could explore for 2 minutes before being re-exposed to the CS. Freezing was analyzed for 1-3 minutes using a FreezeScan video tracking system and software (Cleversys, Inc).

**Y maze.** The Y Maze task was conducted using an established forced alternation protocol(55). During the training phase, mice were placed in the start arm facing the wall and allowed to explore the start and trained arm for 5 minutes, while entry to the 3^{rd} arm (novel arm) was blocked. The maze was cleaned between each mouse to remove odor cues, and the trained arm was alternated between mice. The mouse was then removed to its home cage. After 30 minutes, the block was removed and the mouse was returned to the start arm and allowed to explore all 3 arms for 5 minutes. Time spent in each arm was quantified using the Smart Video Tracking Software (Panlab; Harvard Apparatus). Percent time in each arm was defined as time in arm divided by time spent in all arms during the first minute of the task.

**Novel object recognition.** The novel object recognition task was adapted from a previously described protocol(*56*). During the habituation phase (day 1), mice could freely explore an empty arena for 10 minutes. During the training phase (day 2), two identical objects were placed in the habituated arena, and mice could explore the objects for 5 minutes. For the testing phase (day 3), one object was replaced with a novel object, and mice could explore the objects for 5 minutes. Time spent exploring each object was quantified using the Smart Video Tracking Software (Panlab; Harvard Apparatus). Two different sets of objects are used. To control for any inherent object preference, half of the mice are exposed to object A as their novel object and half to object B. To control for any potential object-independent location preference, the location of the novel object relative to the trained object is also varied. The objects were chosen based on their ability to capture the animal's interest, independent of genetic background or age. To determine percent time with novel object, we calculate (Time with novel object)/(Time with Trained Object + Time with Novel Object) * 100(56). In this preference index, 100% indicates full preference for the novel object, and 0% indicates full preference for the trained object. A mouse with a value of 50% would have spent equal time exploring both objects. Mice that did not explore both objects during the training phase were excluded from analysis.

**Immunohistochemistry.** Tissue processing and immunohistochemistry was performed on free-floating sections following standard published techniques(57). Mice were anesthetized with 87.5 mg/kg ketamine and 12.5mg/kg xylazine and transcardially perfused with phosphate buffered saline. Brains were removed and fixed in phosphate-buffered 4% paraformaldehyde, pH 7.4, at 4C for 48h before cryprotection with 30% sucrose. Brains were sectioned coronally at 40um with a cryomicrotime (Leica Camera, Inc.). Free floating coronal sections (40 µm) were incubated overnight with goat anti-DCX (1:7500, Santa Cruz), mouse anti-NeuN (1:1000, Millipore), rat anti-BrdU (1:1000, Abcam), goat anti-Sox2 (1:200, Santa Cruz), and rabbit anti-GFAP (1:1000, Dako) primary antibodies. Staining was revealed using fluorescent-conjugated secondary antibodies (Life Technologies). For BrdU labeling, brain sections were pre-treated with 2 N HCl at 37 C for 20 min and washed with Tris-buffered saline with Tween (TBST) before primary antibody incubation. Sections were imaged by confocal microscope (Zeiss LSM800). Individual cell number in the dentate gyrus was quantified using ImageJ software.

**RNA extraction, cDNA synthesis and qPCR.** Total RNA was isolated using TRI Reagent (Sigma-Aldrich, cat#T9424). To quantify mRNA expression levels, equal amounts of cDNA were synthesized using the High-Capacity cDNA Reverse Transcription kit (ThermoFisher Scientific, Cat# 4368813), then mixed with SYBR Fast mix (Kapa Biosystems) and primers. GAPDH or actin mRNA was amplified as an internal control. Quantitative RT-PCR was carried out in a CFX384 Real Time System (Bio-Rad).

**Western Blot Analysis.** Mouse hippocampi were dissected after perfusion, snap frozen and lysed in RIPA lysis buffer (Cat# ab156034, Abcam) with complete protease inhibitor (Cat# 4693116001, Sigma-Aldrich) and phosphatase inhibitor (Cat# 78420, Thermo-Fisher). Tissue lysates or plasma were mixed with 4x NuPage LDS loading buffer (Cat# NP0008, Invitrogen), loaded on a 4-12% SDS polyacrylamide gradient gel (Invitrogen) and transferred onto a nitrocellulose membrane. Equal loading of plasma was confirmed using Ponceau S solution (Cat# P7170, Sigma-Aldrich). The blots were blocked in 5% milk in Tris-Buffered Saline with Tween (TBST) and incubated with mouse anti-Tubulin B 3 (1:5000, BioLegend), abbit anti-B-actin (1:1000, Sigma-Aldrich), rabbit anti-BDNF (1:750, Invitrogen), rabbit anti-GPLD1 (1:500, Aviva Systems Biology), rabbit anti-vitronectin (1:500, Molecular Innovations), and rabbit anti-plasminogen (1:1000, Abcam). Horseradish peroxidase-conjugated secondary antibodies and an ECL kit (GE Healthcare) were used to detect protein signals. Membranes were imaged with the ChemiDoc System (BioRad). Selected images were exported and quantified using ImageJ software (Version 2.0.0). Tubulin bands were used for normalization.

**Mass Spectrometry.** For proteomic analysis of exercise plasma, plasma was collected from mature and aged exercised and sedentary mice. Plasma from two mice was pooled for each condition, in duplicate, for a total of 8 samples. Samples were depleted of the most abundant proteins using a MARS3-mouse immunoaffinity column (Agilent) and desalted using Zeba columns (Pierce). Protein concentration was determined using the BCA assay (Pierce). Plasma proteins (50 ug) were digested with trypsin and labeled with iTRAQ 8plex reagent (Applied Biosystems) as previously described(58). Peptides were first separated offline into 18 fractions using alkaline pH reversed-phase HPLC (59). Peptides from each fraction were separated using a nanoLC Ultra 2D Plus system (SCIEX) interfaced with a 5600 Triple TOF mass spectrometer (SCIEX). The peptides were initially loaded onto a guard column (300 µm i.d. × 5 mm, 5-µm particle size, 100-Å pore size; Acclaim PepMap300 C18; Thermo-Fisher) and were washed with the aqueous loading solvent that consisted of 2% solvent B [98% acetonitrile (ACN)/0.1% formic acid (FA)] in solvent A (2% ACN/0.1% FA), flow rate 12 µL/min for 3 min. Then the peptides were separated on a C18 Acclaim PepMap100 column (75 µm i.d. × 150 mm, 3-mm particle size, 100-Å pore size; Thermo Fisher Scientific) heated at 40 °C with a column oven. Peptides were eluted at a flow rate of 300 nL/min with a gradient of 2-35% solvent B for 90 min. In positive ion mode, MS scans from m/z 400-1,600 were acquired followed by MS/MS scans of the 20 most abundant ions with an exclusion time of 15 s. Proteins were identified and quantified using ProteinPilot v.5.0 software and the UniProt v. 201505 database with Mus musculus specied filter and ID focus of biological modifications. iTRAQ ratios were calculated using (one of the pools of old runners, OR1) and bias correction and background subtraction were applied. Protein ratios were calculated using only ratios from the peptide spectra that were distinct to each protein or protein form, thus eliminating any masking of changes in expression caused by the sharing of peptides among proteins. A target-decoy database containing the reversed sequences of all the proteins appended to the target database was used to calculate peptide and protein FDR rates(*60*). A total of 227 proteins were detected at the 5% local FDR.

For proteomic analysis of Gpldl HDTVI plasma, plasma was collected from aged mice 24 hours after HDTVI with either Gpldl or GFP plasmid DNA. Plasma was collected from 3 individual mice per condition for a total of 6 samples. Samples were depleted of the most abundant proteins using Proteome Purify 2 Mouse Serum Protein Immunodepletion Resin (R&D Systems) according to the manufacturer's protocol. Depleted samples were buffer exchanged into water on a Corning Spin X 5kD molecular weight cut off spin column and quantified by Qubit fluorometry (Life Technologies). Plasma proteins (50µg) were reduced with dithiothreitol, alkylated with iodoacetamide and digested with trypsin (Promega). Individual digested samples were processed by solid phase extraction using an Empore C18 (3M) plate under vacuum. Briefly, columns were activated (400µL 95% acetonitrile/0.1% TFA X2) and equilibrated (400µL 0.1% TFA X4). Next, acidified samples were loaded and columns were washed (400µL 0.1% TFA X2). Finally, peptides were eluted (200µL 70% acetonitrile/0.1% TFA X2) and lyophilized for downstream processing. For mass spectrometry analysis, 2µg of protein per sample was analyzed by nano LC-MS/MS with a Waters NanoAcquity interfaced to a ThermoFisher Fusion Lumos mass spectrometer. Peptides were loaded on a trapping column and eluted over a 75 µm × 50cm analytical column (ThermoFisher PIN ES-803) at 300nL/min using a 3 hour reverse phase gradient. The mass spectrometer was operated in data-dependent mode, with the Orbitrap operating at 60,000 FWHM and 15,000 FWHM for MS and MS/MS respectively. The instrument was run with a 3s cycle for MS and MS/MS and APD was enabled. The data were processed with MaxQuant (version 1.6.0.13; Max Planck Institute for Biochemistry(61)), which incorporates the Andromeda search engine. Using this program, the MS data were recalibrated, protein/peptide identification was made using the Andromeda database search engine, the database search results were filtered at the 1% protein and peptide FDR, and protein levels were quantified. The resulting MaxQuant output was further processed using Perseus (V 1.6.0.7; Max Planck Institute for Biochemistry). Gene ontology analysis was performed using STRING(62) on the top 20 up and down-regulated proteins, as sorted by p value.

**Cell Culture.** A HEK 293T cell line was used for *in vitro* experiments. Cells were plated at a confluency of 85-90% for transfection experiments and cultured in DMEM + 10% FBS. Lipofectamine 3000 (ThermoFisher, Cat# L300015) was used as the transfection reagent. Supernatant was collected at 48 hours for downstream analysis. For enzymatic activity of Gpldl, EF1a-Cre and Ubiquitin-Lox-stop-lox-PLAP were co-transfected with CMV-GFP, CMV-Gpld1, CMV-Gpld1-H133A, or CMV-Gpld1-H158A. An SEAP Reporter Gene Assay Kit (Abcam, Cat# 133077) was used to measure alkaline phosphatase activity in the media.

**HDTVI plasmids.** RNA was isolated from adult mouse liver tissue using TRIZol reagent (Thermo Fisher Scientific, Cat# 15596026) and PureLink^{™} RNA Mini Kit following the manufacturer's instructions. The RNA concentration was determined via Nanodrop and RNA was reverse transcribed using the High-Capacity cDNA Reverse Transcription Kit (Thermo Fisher Scientific, Cat# 4368813) and oligo dT primers (Promega cat# C1101). The following primers were used for PCR amplification of the Gpld1 and Pon1 coding sequences and partial 3' and 5' untranslated regions (UTRs) from mouse liver cDNA: CACCGGAGTGACCATCAACTGGCA (Gpldl forward primer); TCAAGAAAGCGGGGCTGAAT (Gpldl reverse primer), CACCAGTGTTGCTGCACT TGTCC (Pon1 forward primer), CACTTTCGATGACGTGCGTG (Pon1 reverse primer). The Gpldl and Pon1 ORFs were cloned into the pENTR D-TOPO vector (ThermoFisher Scientific, Cat# K240020) and sequence verified using the following primers: M13F, M13R, GGAAAGTCATCACCAAAGACGTCC (Gpldl-specific sequencing primer), CAGGGCAGCTCA CCTACAATG (Gpldl-specific sequencing primer). The coding sequences were further amplified and cloned into a mammalian expression plasmid using the restriction sites NheI and EcoRI for Gpldl and XbaI and EcoRI for Pon1. The H133N and H158N mutations were generated using the and the QuikChange Lightning Site-directed Mutagenesis kit (Agilent cat# 210518) in combination with the following primers: H133N Primer 1 (GCTGACGTGAGCTGGAATAGCCTGGGTATTG), H133N Primer 2 (CAATACCCAGGCTATTCCAGCTCACGTCAGC), H158N Primer 1 (TACAACTCTTACTCTGACGCTAACTCGGCTGGTG) H158N Primer 2 (CACCAGCCGAGTTAGCGTCAGAGTAAGAGTTGTA). The bicistronic plasmid vectors expressed Gpldl or Pon1 and an IRES eGFP reporter using a CMV promoter. Empty IRES eGFP constructs based on the same plasmids were used as controls. All coding plasmid sequences were verified by Sanger sequencing. Endotoxin free plasmid kits were used for plasmid preparation prior to in vivo use.

**HiBiT Analysis.** To detect the localization of HiBiT-tagged Gpldl in various mouse tissues, mice were hydrodynamically injected with GFP, Gpldl, or Gpldl-HiBiT constructs. At 24 hours following HDTVI, mice were euthanized and plasma was collected by intracardial bleed. Following perfusion, hippocampus, cortex, cerebellum, and liver were dissected, snap frozen and lysed in RIPA lysis buffer (Abcam, Cat# ab156034) with complete protease inhibitor (Sigma-Aldrich, Cat# 4693116001) and phosphatase inhibitor (Cat# 78420, Thermo-Fisher). 20µg of protein from each sample was loaded in duplicate in an opaque 96 well plate (Corning, Cat# 353296). HiBiT luminescence was measured on the Cytation 5 (BioTek) using the Nano-Glo HiBiT Lytic Detection System (Promega, Cat# N3030) according to the manufacturer's instructions.

**Human Participants.** 20 community-dwelling older adults (66-78 years; 10 F) enrolled in the observational UCSF Memory and Aging Center Hillblom Study of healthy brain aging were selected for inclusion. Inclusion criteria were: 1) no diagnosed memory or neurological condition (e.g., epilepsy, stroke), 2) no functional decline operationalized as a Clinical Dementia Rating scale of 0 (via study partner interviews), 3) blood draw at study visit, and 4) completion of physical activity monitoring for ≥2 weeks following study visit. To achieve optimal activity variability and blinded to all other outcomes, we selected participants aged 60-80 with the highest (5 female, 5 male) and lowest (5 female, 5 male) step-counts to be included in the current study. The study was approved by the UCSF IRB on human research and all participants provided written, informed consent before enrolling. The study followed the guidelines of the Helsinki declaration. A summary table of participant characteristics is below.

**Table 1. Summary of participant characteristics**

| *Mean (SD)* | | |
|---|---|---|
| **Age** | | 73.7 (3.3) |
| **Sex, % F (n)** | | 50% (10) |
| **Education** | | 18.2 (1.9) [range: 13, 20] |
| **Fitbit Flex2 Daily Steps** | | 8565.01 (4483.1) [range: 930.5, 16270.3] |
| **Days monitored** | | 31.4 (4.2) [range: 19, 39] |
| **BMI** | | 26.6 (5.9) [range: 19.8, 46.7] |
| **Height** | | 66.6 (4.9) [range: 57, 75] |
| **Weight** | | 166.5 (33.0) [range: 111, 220] |
| **Heart Rate** | | 65.5 (8.8) [range: 48, 85] |
| **Blood Pressure** | | |
| | **Systolic** | 130.9 (17.0) [range: 100-172] |
| | **Diastolic** | 71.4 (7.9) [range: 58, 85] |
| | | |

**Human Physical Activity Measurement.** Fitbit Flex2^{™} accelerometers (Fitbit Inc., San Francisco, CA) were used to objectively capture physical activity levels. Participants were instructed to wear the waterproof, triaxial accelerometers on their nondominant wrist during all waking hours. To minimize motivational factors on observational activity data, device feedback was removed (i.e., all informational tiles on phone app deleted and daily "goal" on all devices was set to an unachievable level - 1 million steps) and participants were instructed to not change app settings. Participants were instructed to sync and charge devices each night. Each Fitbit account was linked to the Fitabase platform (Small Steps Labs, LLC, San Diego, CA), which retrieved data from Fitbit API and aggregated daily step counts. Days with <300 steps were removed for quality control, per published protocols (*63*) and daily steps were averaged over the monitoring period (*M* = 31.4 days, range: 19-39 days). Participants were dichotomized into "active" versus "sedentary" using average daily step ≥7100, consistent with guidelines for older adults(*64*).

**Data and statistical analyses.** All experiments were randomized and blinded by an independent researcher before plasma or hydrodynamic tail vein injection. Researchers remained blinded throughout histological, biochemical and behavioral assessments. Groups were un-blinded at the end of each experiment upon statistical analysis. Data are expressed as mean ± s.e.m. The distribution of data in each set of experiments was tested for normality using D'Agostino-Pearson omnibus test or Shapiro-Wilk test. Statistical analysis was performed with Prism 8.0 software (GraphPad Software). Means between two groups were compared with two-tailed, unpaired Student's *t*- test. Comparisons of means from multiple groups with each other were analyzed with one-way ANOVA followed by Tukey's *post hoc* test. Trial by group interactions were analyzed using repeated measures ANOVA and Bonferroni *post hoc* test.

### References cited in application by citation number:

1. K. I. Erickson et al., Exercise training increases size of hippocampus and improves memory. Proceedings of the National Academy of Sciences of the United States of America. 108, 3017-3022 (2011).
2. A. Maass et al., Vascular hippocampal plasticity after aerobic exercise in older adults. Mol. Psychiatry. 20, 585-593 (2015).
3. A. M. Horowitz, S. A. Villeda, Therapeutic potential of systemic brain rejuvenation strategies for neurodegenerative disease. F1000Res. 6, 1291 (2017).
4. M. P. Mattson, T. Magnus, Ageing and neuronal vulnerability. Nature Reviews Neuroscience. 7, 278-294 (2006).
5. M. Mather, L. A. Jacobsen, K. M. Pollard, Aging in the United States. 70 (2015).
6. A. Association, 2016 Alzheimer's Disease Facts and Figures. 12 (2016).
7. R. E. Rhodes et al., Factors associated with exercise adherence among older adults. An individual perspective. Sports Med. 28, 397-411 (1999).
8. H. van Praag, T. Shubert, C. Zhao, F. H. Gage, Exercise enhances learning and hippocampal neurogenesis in aged mice. J. Neurosci. 25, 8680-8685 (2005).
9. A. Kumar, A. Rani, O. Tchigranova, W.-H. Lee, T. C. Foster, Influence of late-life exposure to environmental enrichment or exercise on hippocampal function and CA1 senescent physiology. Neurobiol. Aging. 33, 828.e1-17 (2012).
10. R. B. Speisman, A. Kumar, A. Rani, T. C. Foster, B. K. Ormerod, Daily exercise improves memory, stimulates hippocampal neurogenesis and modulates immune and neuroimmune cytokines in aging rats. Brain Behav. Immun. 28, 25-43 (2013).
11. I. Soto et al., APOE Stabilization by Exercise Prevents Aging Neurovascular Dysfunction and Complement Induction. 13, e1002279 (2015).
12. X. Fan, E. G. Wheatley, S. A. Villeda, Mechanisms of Hippocampal Aging and the Potential for Rejuvenation. Annu. Rev. Neurosci. 40, annurev-neuro-072116-031357 (2017).
13. S. A. Villeda et al., The ageing systemic milieu negatively regulates neurogenesis and cognitive function. 477, 90-94 (2011).
14. S. A. Villeda et al., Young blood reverses age-related impairments in cognitive function and synaptic plasticity in mice. Nat. Med. 20, 659-663 (2014).
15. L. Katsimpardi et al., Vascular and neurogenic rejuvenation of the aging mouse brain by young systemic factors. Science. 344, 630-634 (2014).
16. J. M. Castellano et al., Human umbilical cord plasma proteins revitalize hippocampal function in aged mice. Nature. 544, 488-492 (2017).
17. L. Khrimian et al., Gpr158 mediates osteocalcin's regulation of cognition. J. Exp. Med. 214, 2859-2873 (2017).
18. G. Gontier et al., Tet2 Rescues Age-Related Regenerative Decline and Enhances Cognitive Function in the Adult Mouse Brain. Cell reports. 22, 1974-1981 (2018).
19. C.-W. Wu et al., Exercise enhances the proliferation of neural stem cells and neurite growth and survival of neuronal progenitor cells in dentate gyrus of middle-aged mice. J Appl Physiol. 105, 1585-1594 (2008).
20. H. van Praag, B. R. Christie, T. J. Sejnowski, F. H. Gage, Running enhances neurogenesis, learning, and long-term potentiation in mice. Proceedings of the National Academy of Sciences of the United States of America. 96, 13427-13431 (1999).
21. S. Lugert et al., Quiescent and Active Hippocampal Neural Stem Cells with Distinct Morphologies Respond Selectively to Physiological and Pathological Stimuli and Aging. Cell Stem Cell. 6, 445-456 (2010).
22. D. J. Creer, C. Romberg, L. M. Saksida, H. van Praag, T. J. Bussey, Running enhances spatial pattern separation in mice. Proceedings of the National Academy of Sciences of the United States of America. 107, 2367-2372 (2010).
23. Tabula Muris Consortium et al., Single-cell transcriptomics of 20 mouse organs creates a Tabula Muris. Nature. 562, 367-372 (2018).
24. M. Uhlén et al., Proteomics. Tissue-based map of the human proteome. Science. 347, 1260419-1260419 (2015).
25. B. J. Scallon et al., Primary structure and functional activity of a phosphatidylinositol-glycan-specific phospholipase D. Science. 252, 446-448 (1991).
26. G. A. Maguire, A. Gossner, Glycosyl phosphatidyl inositol phospholipase D activity in human serum. Ann. Clin. Biochem. 32 ( Pt 1), 74-78 (1995).
27. M. K. Schwinn et al., CRISPR-Mediated Tagging of Endogenous Proteins with a Luminescent Peptide. ACS Chem. Biol. 13, 467-474 (2018).
28. M. A. Davitz et al., A glycan-phosphatidylinositol-specific phospholipase D in human serum. Science. 238, 81-84 (1987).
29. C. N. Metz et al., Release of GPI-anchored membrane proteins by a cell-associated GPI-specific phospholipase D. EMBO J. 13, 1741-1751 (1994).
30. Y. Fujihara, M. Ikawa, GPI-AP release in cellular, developmental, and reproductive biology. Journal of lipid research. 57, 538-545 (2016).
31. M. G. Low, A. R. S. Prasad, A phospholipase D specific for the phosphatidylinositol anchor of cell-surface proteins is abundant in plasma. Proceedings of the National Academy of Sciences. 85, 980-984 (1988).
32. M. Del Rosso et al., The urokinase receptor system, a key regulator at the intersection between inflammation, immunity, and coagulation. Curr. Pharm. Des. 17, 1924-1943 (2011).
33. F. Blasi, N. Sidenius, The urokinase receptor: focused cell surface proteolysis, cell adhesion and signaling. FEBS Lett. 584, 1923-1930 (2010).
34. N. S. Raikwar, R. F. Bowen, M. A. Deeg, Mutating His29, His125, His133 or His158 abolishes glycosylphosphatidylinositol-specific phospholipase D catalytic activity. Biochem. J. 391, 285-289 (2005).
35. S. F. Sorrells et al., Human hippocampal neurogenesis drops sharply in children to undetectable levels in adults. Nature. 555, 377 EP -.
36. E. P. Moreno-Jiménez et al., Adult hippocampal neurogenesis is abundant in neurologically healthy subjects and drops sharply in patients with Alzheimer's disease. Nature medicine. 25, 554-560 (2019).
37. M. K. Tobin et al., Human Hippocampal Neurogenesis Persists in Aged Adults and Alzheimer's Disease Patients. Cell Stem Cell (2019), doi:10.1016/j.stem.2019.05.003.
38. N. T. Lautenschlager et al., Effect of physical activity on cognitive function in older adults at risk for Alzheimer disease: a randomized trial. JAMA. 300, 1027-1037 (2008).
39. Y. E. Geda et al., Physical exercise, aging, and mild cognitive impairment: a population-based study. Arch. Neurol. 67, 80-86 (2010).
40. S. Müller et al., Relationship between physical activity, cognition, and Alzheimer pathology in autosomal dominant Alzheimer's disease. Alzheimers Dement. 14, 1427-1437 (2018).
41. K. A. Intlekofer, C. W. Cotman, Exercise counteracts declining hippocampal function in aging and Alzheimer's disease. Neurobiology of disease (2013).
42. S. H. Choi et al., Combined adult neurogenesis and BDNF mimic exercise effects on cognition in an Alzheimer's mouse model. Science. 361, eaan8821 (2018).
43. L. K. Smith et al., β2-microglobulin is a systemic pro-aging factor that impairs cognitive function and neurogenesis. Nat. Med. 21, 932-937 (2015).
44. J. Rebo et al., A single heterochronic blood exchange reveals rapid inhibition of multiple tissues by old blood. Nat Commun. 7, 13363 (2016).
45. H. Yousef et al., Aged blood impairs hippocampal neural precursor activity and activates microglia via brain endothelial cell VCAM1. Nature medicine. 25, 988-1000 (2019).
46. C. D. Wiley et al., SILAC Analysis Reveals Increased Secretion of Hemostasis-Related Factors by Senescent Cells. Cell reports. 28, 3329-3337.e5 (2019).
47. A. T. Naito et al., Complement C1q activates canonical Wnt signaling and promotes aging-related phenotypes. Cell. 149, 1298-1313 (2012).
48. S. K. Baker et al., Blood-derived plasminogen drives brain inflammation and plaque deposition in a mouse model of Alzheimer's disease. Proceedings of the National Academy of Sciences of the United States of America. 115, E9687-E9696 (2018).
49. M. A. Shaw et al., Plasminogen Deficiency Delays the Onset and Protects from Demyelination and Paralysis in Autoimmune Neuroinflammatory Disease. J. Neurosci. 37, 3776-3788 (2017).
50. I. M. Conboy et al., Rejuvenation of aged progenitor cells by exposure to a young systemic environment. Nature. 433, 760-764 (2005).
51. M. Sinha et al., Restoring systemic GDF11 levels reverses age-related dysfunction in mouse skeletal muscle. Science. 344, 649-652 (2014).
52. G. S. Baht et al., Exposure to a youthful circulaton rejuvenates bone repair through modulation of β-catenin. Nat Commun. 6, 7131 (2015).
53. D. Kovacsics, J. Paper, Transient expression of proteins by hydrodynamic gene delivery in mice. J Vis Exp (2014), doi:10.3791/51481.
54. J. Alamed, D. M. Wilcock, D. M. Diamond, M. N. Gordon, D. Morgan, Two-day radial-arm water maze learning and memory task; robust resolution of amyloid-related memory deficits in transgenic mice. Nat Protoc. 1, 1671-1679 (2006).
55. K. Belarbi et al., Beneficial effects of exercise in a transgenic mouse model of Alzheimer's disease-like Tau pathology. Neurobiology of disease. 43, 486-494 (2011).
56. D. B. Dubal et al., Life extension factor klotho prevents mortality and enhances cognition in hAPP transgenic mice. J. Neurosci. 35, 2358-2371 (2015).
57. L. K. Smith et al., β2-microglobulin is a systemic pro-aging factor that impairs cognitive function and neurogenesis. Nat. Med. 21, 932-937 (2015).
>Qu K. E. Williams et al., Quantitative proteomic analyses of mammary organoids reveals distinct signatures after exposure to environmental chemicals. Proceedings of the National Academy of Sciences of the United States of America. 113, E1343-51 (2016).
59. R. C. Dwivedi et al., Practical implementation of 2D HPLC scheme with accurate peptide retention prediction in both dimensions for high-throughput bottom-up proteomics. Anal. Chem. 80, 7036-7042 (2008).
60. W. H. Tang, I. V. Shilov, S. L. Seymour, Nonlinear fitting method for determining local false discovery rates from decoy database searches. J. Proteome Res. 7, 3661-3667 (2008).
61. S. Tyanova, T. Temu, J. Cox, The MaxQuant computational platform for mass spectrometry-based shotgun proteomics. Nat Protoc. 11, 2301-2319 (2016).
62. D. Szklarczyk et al., STRING v11: protein-protein association networks with increased coverage, supporting functional discovery in genome-wide experimental datasets. Nucleic Acids Res. 47, D607-D613 (2019).
63. V. J. Block et al., Continuous daily assessment of multiple sclerosis disability using remote step count monitoring. J. Neurol. 264, 316-326 (2017).
64. C. Tudor-Locke et al., How many steps/day are enough? For older adults and special populations. International Journal of Behavioral Nutrition and Physical Activity. 8, 80 (2011).

### Illustrative Sequences

**SEQ ID NO:1 UniProtKB Accession No. P80108 human Gpld1 isotype 1 ("canonical" sequence); signal peptide position 1-23; mature peptide positions 24-840** 
MSAFRLWPGL LIMLGSLCHR GSP CGLSTHV EIGHRALEFL QLHNGRVNYR

ELLLEHQDAY QAGIVFPDCF YPSICKGGKF HDVSESTHWT PFLNASVHYI

RENYPLPWEK DTEKLVAFLF GITSHMAADV SWHSLGLEQG FLRTMGAIDF

HGSYSEAHSA GDFGGDVLSQ FEFNFNYLAR RWYVPVKDLL GIYEKLYGRK

VITENVIVDC SHIQFLEMYG EMLAVSKLYP TYSTKSPFLV EQFQEYFLGG

LDDMAFWSTN IYHLTSFMLE NGTSDCNLPE NPLFIACGGQ QNHTQGSKMQ

KNDFHRNLTT SLTESVDRNI NYTERGVFFS VNSWTPDSMS FIYKALERNI

RTMFIGGSQL SQKHVSSPLA SYFLSFPYAR LGWAMTSADL NQDGHGDLVV

GAPGYSRPGH IHIGRVYLIY GNDLGLPPVD LDLDKEAHRI LEGFQPSGRF

GSALAVLDFN VDGVPDLAVG APSVGSEQLT YKGAVYVYFG SKQGGMSSSP

NITISCQDIY CNLGWTLLAA DVNGDSEPDL VIGSPFAPGG GKQKGIVAAF

YSGPSLSDKE KLNVEAANWT VRGEEDFSWF GYSLHGVTVD NRTLLLVGSP

TWKNASRLGH LLHIRDEKKS LGRVYGYFPP NGQSWFTISG DKAMGKLGTS

LSSGHVLMNG TLKQVLLVGA PTYDDVSKVA FLTVTLHQGG ATRMYALTSD

AQPLLLSTFS GDRRFSRFGG VLHLSDLDDD GLDEIIMAAP LRIADVTSGL

IGGEDGRVYV YNGKETTLGD MTGKCKSWIT PCPEEKAQYV LISPEASSRF

GSSLITVRSK AKNQVVIAAG RSSLGARLSG ALHVYSLGSD
**SEQ ID NO:2 UniProtKB Accession No. O70362 mouse Gpld1 polypeptide; signal peptide position 1-23; mature peptide positions 24-837**

MSAGRLWSSL LLLLPLFCSK SSS CGLSTHV EIGHRALEFL RLQDGRINYK

ELILEHQDAY QAGTVFPDAF YPSICKRGKY HDVSERTHWT PFLNASIHYI

RENYPLPWEK DTEKLVAFLF GITSHMVADL SWHNLGFLRT MGAIDFYNSY
SDAHSAGDFG GDVLSQFEFN FNYLSRRWYV PVRDLLRIYD NLYGRKVITK
DVLVDCTYLQ FLEMHGEMFA VSKLYSTYST KSPFLVEQFQ DYFLGGLDDM
AFWSTNIYRL TSFMLENGTS DCNLPENPLF ISCDGRNHTL SGSKVQKNDF
HRNLTMFISR DIRKNLNYTE RGVFYSTGSW ARPESVTFMY QTLERNLRLM
LAGSSQKNLN HVSSPSASYT LSVPYARLGW VMTSADLNQD GHGDLVVGAP
GYSHPGRFQI GRVYIIYGND LGLPPIDLDL NKEGILEGFQ PSGRFGSALA
VLDFNQDGLP DLAVGAPSVG SGQLTYNGSV YVYYGSQQGR LSSSPNVTIS
CKDTYCNLGW TLLATDADGD GRHDLVISSP FAPGGRKQKG IVATFYSHPR
RNDKELLTLE EADWKVNGEE DFSWFGYSLH GVTVANRSLL LIGSPTWKNV
SRMARSSHKK NQEEKSLGKV YGYFLPNRQS TITISGDKAM GKLGTSLSSG
YVRVNGTLTQ VLLVGAPTHD DVSKMAFLTM TLHQGGATRM YELAPEKTQP
ALLSTFSGDR RFSRFGSVLH LTDLDDDGLD EIIMAAPLRI TDVTSGLLGG
EDGRVYIYNG MYTTLGDMTG KCKSWMTPCP EEKAQYVLTS PEASSRFGSS
LVSVRSKGRN QVVVAAGRSS WGARLSGALH VYSFSSD

## Claims

1. A phosphatidylinositol-glycan-specific phospholipase D1 (Gpld1) polypeptide that comprises the catalytic domain and has catalytic activity for use in a method of treating or preventing age-related cognitive dysfunction in an individual in need thereof, wherein the method comprises
administering to the individual an effective amount of the Gpld1 polypeptide systemically or locally to the brain,
thereby treating or preventing age-related cognitive dysfunction.

2. The Gpld1 polypeptide for use according to claim 1, wherein the Gpld1 polypeptide is administered systemically.

3. The Gpld1 polypeptide for use according to claim 1, wherein the Gpld1 polypeptide is administered by intravenous, intraperitoneal, subcutaneous, or intramuscular injection.

4. The Gpld1 polypeptide for use according to claim 1, wherein the Gpld1 polypeptide is administered orally or mucosally.

5. The Gpld1 polypeptide for use according to any one of claims 1 to 4, wherein the individual is a human,
optionally wherein
(i) the human is at least 50 years old;
(ii) the individual has mild cognitive impairment;
(iii) the individual has dementia; or
(iv) the individual has Alzheimer's disease.

6. The Gpld1 polypeptide for use according to any one of claims 1 to 5, wherein
a) the Gpld1 polypeptide has catalytic activity and comprises an amino acid sequence having at least 75% identity to amino acids 24-176 of SEQ ID NO: 1,
optionally wherein the amino acid sequence has at least 95% identity to amino acids 24-176 of SEQ ID NO:1; or
b) the Gpld1 polypeptide has catalytic activity and comprises an amino acid sequence having at least 75%, at least 80%, at least 90%, or at least 95% identity to the region of SEQ ID NO:1 that corresponds to the mature protein.

7. The Gpld1 polypeptide for use according to any one of the foregoing claims, wherein the effective amount is 1 µg to 1000 µg per kg body weight of the individual.

8. The Gpld1 polypeptide for use according to any one of the foregoing claims, wherein the Gpld1 polypeptide is administered more than once as part of a course of treatment.

9. The Gpld1 polypeptide for use according to any one of the foregoing claims, wherein the method of treating or preventing age-related cognitive dysfunction in the individual in need thereof further comprises testing the cognitive function of the individual after administering to the individual an effective amount of the Gpld1 polypeptide,
optionally wherein the method further comprises testing the cognitive function of the individual prior to administering and comparing the cognitive function of the individual prior to and after administering.

10. The Gpld1 polypeptide for use according to claim 9, wherein
a) cognitive function is determined by testing the individual for semantic, episodic, procedural, priming, and/or working memory; or
b) cognitive function is determined by testing the individual for language ability, executive function, visuospatial function, or dementia.

11. A nucleic acid construct encoding a Gpld1 polypeptide for use in a method of treating or preventing age-related cognitive dysfunction in an individual in need thereof, the method comprising introducing the nucleic acid construct into the patient,
optionally wherein the nucleic acid is introduced into the liver.

12. Genetically modified cells of an individual for use in a method of treating or preventing age-related cognitive dysfunction in the individual, the method comprising genetically modifying the cells *ex vivo* to enhance expression of a Gpld1 polypeptide using a genetic modification technique and administering the cells to the individual, optionally wherein the genetic modification technique is CRISPR/Cas or by introducing a nucleic acid construct encoding a Gpld1 polypeptide into the cells.

13. The genetically modified cells for use according to claim 12, wherein the cells are hepatocytes.

14. The genetically modified cells for use according to claim 12 or 13, wherein
a) the individual has mild cognitive impairment;
b) the individual has dementia; or
c) the individual has Alzheimer's disease.

## Patentansprüche

1. Phosphatidylinositol-Glykan-spezifisches Phospholipase D1- (Gpld1) Polypeptid, das die katalytische Domäne umfasst und katalytische Aktivität zur Verwendung in einem Verfahren zur Behandlung oder Vorbeugung altersbedingter kognitiver Dysfunktion bei einem Individuum, das diese benötigt, aufweist, wobei das Verfahren umfasst:
Verabreichen einer wirksamen Menge des Gpld1-Polypeptids an das Individuum systemisch oder lokal an das Gehirn,
wodurch altersbedingte kognitive Dysfunktion behandelt oder verhindert wird.

2. Gpld1-Polypeptid zur Verwendung nach Anspruch 1, wobei das Gpld1-Polypeptid systemisch verabreicht wird.

3. Gpld1-Polypeptid zur Verwendung nach Anspruch 1, wobei das Gpld1-Polypeptid durch intravenöse, intraperitoneale, subkutane oder intramuskuläre Injektion verabreicht wird.

4. Gpld1-Polypeptid zur Verwendung nach Anspruch 1, wobei das Gpld1-Polypeptid oral oder mukosal verabreicht wird.

5. Gpld1-Polypeptid zur Verwendung nach einem der Ansprüche 1 bis 4, wobei das Individuum ein Mensch ist,
optional wobei:
(i) der Mensch mindestens 50 Jahre alt ist;
(ii) das Individuum eine leichte kognitive Beeinträchtigung hat;
(iii) das Individuum Demenz hat; oder
(iv) das Individuum die Alzheimer-Krankheit hat.

6. Gpld1-Polypeptid zur Verwendung nach einem der Ansprüche 1 bis 5, wobei
a) das Gpld1-Polypeptid katalytische Aktivität besitzt und eine Aminosäuresequenz umfasst, die mindestens 75% Identität mit den Aminosäuren 24-176 von SEQ ID NO: 1 aufweist,
wobei optional die Aminosäuresequenz mindestens 95% Identität mit den Aminosäuren 24-176 von SEQ ID NO: 1 aufweist; oder
b) das Gpld1-Polypeptid katalytische Aktivität aufweist und eine Aminosäuresequenz umfasst, die mindestens 75%, mindestens 80%, mindestens 90% oder mindestens 95% Identität mit der Region von SEQ ID NO:1 aufweist, die dem reifen Protein entspricht.

7. Gpld1-Polypeptid zur Verwendung nach einem der vorangehenden Ansprüche, wobei die wirksame Menge 1 µg bis 1000 µg pro kg Körpergewicht des Individuums beträgt.

8. Gpld1-Polypeptid zur Verwendung nach einem der vorangehenden Ansprüche, wobei das Gpld1-Polypeptid mehr als einmal als Teil eines Behandlungsverlaufs verabreicht wird.

9. Gpld1-Polypeptid zur Verwendung nach einem der vorangehenden Ansprüche, wobei das Verfahren zur Behandlung oder Vorbeugung einer altersbedingten kognitiven Dysfunktion bei dem Individuum, das dessen bedarf, ferner das Testen der kognitiven Funktion des Individuums nach Verabreichung einer wirksamen Menge des Gpld1-Polypeptids an das Individuum umfasst,
wobei das Verfahren optional ferner das Testen der kognitiven Funktion des Individuums vor der Verabreichung und das Vergleichen der kognitiven Funktion des Individuums vor und nach der Verabreichung umfasst.

10. Gpld1-Polypeptid zur Verwendung gemäß Anspruch 9, wobei
a) die kognitive Funktion bestimmt wird, indem das Individuum auf semantisches, episodisches, prozedurales, Priming- und/oder Arbeits-Gedächtnis getestet wird; oder
b) die kognitive Funktion bestimmt wird, indem das Individuum auf Sprachfähigkeit, exekutive Funktion, visuell-räumliche Funktion oder Demenz getestet wird.

11. Nukleinsäurekonstrukt, das für ein Gpld1-Polypeptid codiert, zur Verwendung in einem Verfahren zur Behandlung oder Vorbeugung altersbedingter kognitiver Dysfunktion bei einem Individuum, das dies benötigt, wobei das Verfahren das Einführen des Nukleinsäurekonstrukts in den Patienten umfasst,
wobei optional die Nukleinsäure in die Leber eingebracht wird.

12. Genetisch modifizierte Zellen eines Individuums zur Verwendung in einem Verfahren zur Behandlung oder Vorbeugung altersbedingter kognitiver Dysfunktion bei dem Individuum, wobei das Verfahren die genetische Modifikation der Zellen ex vivo zur Verstärkung der Expression eines Gpld1-Polypeptids unter Verwendung einer genetischen Modifikationstechnik und die Verabreichung der Zellen an das Individuum umfasst, wobei es sich bei der genetischen Modifikationstechnik optional um CRISPR/Cas oder um die Einführung eines Nukleinsäurekonstrukts, das für ein Gpld1-Polypeptid codiert, in die Zellen handelt.

13. Genetisch modifizierte Zellen zur Verwendung nach Anspruch 12, wobei die Zellen Hepatozyten sind.

14. Genetisch modifizierte Zellen zur Verwendung nach Anspruch 12 oder 13, wobei
a) das Individuum eine leichte kognitive Beeinträchtigung hat;
b) das Individuum Demenz hat; oder
c) das Individuum die Alzheimer-Krankheit hat.

## Revendications

1. Polypeptide de phospholipase D1 spécifique de phosphatidylinositolglycane (Gpld1) qui comprend le domaine catalytique et a une activité catalytique, pour utilisation dans une méthode de traitement ou de prévention de dysfonction cognitive liée à l'âge chez un individu en ayant besoin, dans lequel la méthode comprend
l'administration, à l'individu, d'une quantité efficace du polypeptide Gpld1 par voie systémique ou locale au cerveau,
ainsi le traitement ou la prévention de dysfonction cognitive liée à l'âge.

2. Polypeptide Gpld1 pour utilisation selon la revendication 1, dans lequel le polypeptide Gpld1 est administré par voie systémique.

3. Polypeptide Gpld1 pour utilisation selon la revendication 1, dans lequel le polypeptide Gpld1 est administré par injection intraveineuse, intrapéritonéale, souscutanée, ou intramusculaire.

4. Polypeptide Gpld1 pour utilisation selon la revendication 1, dans lequel le polypeptide Gpld1 est administré par voie orale ou muqueuse.

5. Polypeptide Gpld1 pour utilisation selon l'une quelconque des revendications 1 à 4, dans lequel l'individu est un humain,
optionnellement dans lequel
(i) l'humain a au moins 50 ans ;
(ii) l'individu a un trouble cognitif léger ;
(iii) l'individu a une démence ; ou
(iv) l'individu a la maladie d'Alzheimer.

6. Polypeptide Gpld1 pour utilisation selon l'une quelconque des revendications 1 à 5, dans lequel
a) le polypeptide Gpld1 a une activité catalytique et comprend une séquence d'acides aminés ayant au moins 75 % d'identité avec les acides aminés 24-176 de SEQ ID n° : 1,
optionnellement dans lequel la séquence d'acides aminés a au moins 95 % d'identité avec les acides aminés 24-176 de SEQ ID n° : 1 ; ou
b) le polypeptide Gpld1 a une activité catalytique et comprend une séquence d'acides aminés ayant au moins 75 %, au moins 80 %, au moins 90 %, ou au moins 95 % d'identité avec la région de SEQ ID n° : 1 qui correspond à la protéine mature.

7. Polypeptide Gpld1 pour utilisation selon l'une quelconque des revendications précédentes, dans lequel la quantité efficace est de 1 µg à 1 000 µg par kg de poids corporel de l'individu.

8. Polypeptide Gpld1 pour utilisation selon l'une quelconque des revendications précédentes, dans lequel le polypeptide Gpld1 est administré plus d'une fois en tant que partie d'un traitement.

9. Polypeptide Gpld1 pour utilisation selon l'une quelconque des revendications précédentes, dans lequel la méthode de traitement ou de prévention de dysfonction cognitive liée à l'âge chez l'individu en ayant besoin comprend en outre la réalisation de tests de la fonction cognitive de l'individu après l'administration, à l'individu, d'une quantité efficace du polypeptide Gpld1,
optionnellement dans lequel la méthode comprend en outre la réalisation de tests de la fonction cognitive de l'individu avant l'administration, et la comparaison de la fonction cognitive de l'individu avant et après l'administration.

10. Polypeptide Gpld1 pour utilisation selon la revendication 9, dans lequel
a) la fonction cognitive est déterminée en soumettant l'individu à des tests de mémoire sémantique, épisodique, procédurale, primaire, et/ou de travail ; ou
b) la fonction cognitive est déterminée en soumettant l'individu à des tests d'aptitude linguistique, de fonction exécutive, de fonction visuo-spatiale, ou de démence.

11. Construction d'acide nucléique codant un polypeptide Gpld1 pour utilisation dans une méthode de traitement ou de prévention de dysfonction cognitive liée à l'âge chez un individu en ayant besoin, la méthode comprenant l'introduction de la construction d'acide nucléique dans le patient,
optionnellement dans laquelle l'acide nucléique est introduit dans le foie.

12. Cellules génétiquement modifiées d'un individu pour utilisation dans une méthode de traitement ou de prévention de dysfonction cognitive liée à l'âge chez l'individu, la méthode comprenant la modification génétique des cellules *ex vivo* pour améliorer l'expression d'un polypeptide Gpld1 en utilisant une technique de modification génétique, et l'administration des cellules à l'individu, optionnellement dans lesquelles la technique de modification génétique est CRISPR/Cas ou en introduisant, dans les cellules, une construction d'acide nucléique codant un polypeptide Gpld1.

13. Cellules génétiquement modifiées pour utilisation selon la revendication 12, dans lesquelles les cellules sont des hépatocytes.

14. Cellules génétiquement modifiées pour utilisation selon la revendication 12 ou 13, dans lesquelles
a) l'individu a un trouble cognitif léger ;
b) l'individu a une démence ; ou
c) l'individu a la maladie d'Alzheimer.
